# FASCICULE DE BREVET EUROPEEN

(11) **EP 2 761 005 B1**
(45) Date de publication et mention de la délivrance du brevet: **20.06.2018**
(21) Numéro de dépôt: 12766082.7
(22) Date de dépôt: 26.09.2012
(51) Int. Cl.: C12N 15/54, C12N 9/10, C12N 15/63, C12R 1/89, C12P 7/22

(54) **UTILISATION DE " POLYKETIDE SYNTHASES " DE TYPE III (PKS III) RECOMBINANTES D'ALGUES BRUNES MARINES**
VERWENDUNG VON REKOMBINANTEN TYP-III-POLYKETID-SYNTHASEN (PKS) AUS MEERESBRAUNALGEN
USE OF RECOMBINANT TYPE III "POLYKETIDE SYNTHASES" (PKS III) OF MARINE BROWN ALGAE

(30) Priorité: 29.09.2011 FR 1158728
(43) Date de publication de la demande: 06.08.2014
(73) Titulaire: Université Pierre et Marie Curie (Paris 6), 75005 Paris (FR); Université de Bretagne Occidentale, 29200 Brest (FR)
(72) Inventeur: DELAGE, Ludovic, F-Mespaul 29420 (FR); MESLET-CLADIERE, Laurence, 29250 Saint-Pol-De-Leon (FR); POTIN, Philippe, F-29680 Roscoff (FR); GOULITQUER, Sophie, F-29200 Brest (FR)
(74) Mandataire: Lavoix
(86) Numéro de dépôt international: PCT/EP2012/068994
(87) Numéro de publication internationale: WO 2013/045510

(56) Documents cités:
- , 22 novembre 2010 (2010-11-22), XP055021037, Extrait de l'Internet: URL:http://genoweb1.irisa.fr/OGP/ftp/Prese ntations/Presentation_Sophie_GOULITQUER.pd f [extrait le 2012-03-06]
- J. MARK COCK ET AL: "The Ectocarpus genome and the independent evolution of multicellularity in brown algae", NATURE, vol. 465, no. 7298, 3 juin 2010 (2010-06-03), pages 617-621, XP055020932, ISSN: 0028-0836, DOI: 10.1038/nature09016
- INDER PAL SINGH ET AL: "Phloroglucinol compounds of therapeutic interest: global patent and technology status", EXPERT OPINION ON THERAPEUTIC PATENTS, vol. 19, no. 6, 1 juin 2009 (2009-06-01), pages 847-866, XP055021032, ISSN: 1354-3776, DOI: 10.1517/13543770902916614 cité dans la demande
- HARIYANTI BAHARUM ET AL: "Molecular Cloning, Modeling, and Site-Directed Mutagenesis of Type III Polyketide Synthase from(Phaeophyta)", MARINE BIOTECHNOLOGY, SPRINGER-VERLAG, NE, vol. 13, no. 5, 23 décembre 2010 (2010-12-23), pages 845-856, XP019939493, ISSN: 1436-2236, DOI: 10.1007/S10126-010-9344-5
- Philippe Potin: ""Algal genomics may offer new insights into seaweed uses "", , 7 September 2010 (2010-09-07), XP55284161, Retrieved from the Internet: URL:http://www.tangbog.dk/wp-content/uploa ds/potin-22.pdf [retrieved on 2016-06-28]

## Description

La présente invention concerne une méthode de production de composés polyphénoliques, *i.e.* le phloroglucinol ou l'un de ses dérivés, par une polyketide synthase de type III (PKSIII) d'algue brune marine. L'invention concerne également des acides nucléiques recombinants codant pour une polyketide synthase de type III (PKSIII) de l'algue brune *Ectocarpus siliculosus* (*E. siliculosus*), des vecteurs recombinants comprenant ces acides nucléiques, ainsi que des cellules hôtes comprenant ces vecteurs. Enfin, l'invention concerne une méthode de préparation de divers composés à l'aide des composés polyphénoliques produits selon la méthode précitée.

Les métabolites secondaires polyphénoliques forment un large groupe de composés chimiques divers qui existent à la fois chez les plantes terrestres et chez les macrophytes aquatiques (Waterman et Mole, 1994, Analysis of phenolic plant metabolites, Blackwell Scientific Publications : Oxford, Great Brita*in*). Parmi ces composés, le phloroglucinol ainsi que ses dérivés sont largement utilisés dans l'industrie, et notamment dans les industries pharmaceutique, cosmétique ou encore agroalimentaire. Plus récemment, le phloroglucinol et ses dérivés ont montré des activités potentiellement intéressantes pour l'homme en pharmacologie (production d'intermédiaires réactionnels pour l'hémi-synthèse de composés chimiques), médecine (propriétés antimicrobiennes, anti-VIH, anti-cancéreux, anti-diabétique, anti-allergique, anti-inflammatoire) ou cosmétique (effet anti-âge) qui en font des molécules naturelles à potentiel très intéressant (Singh IP et al., 2009, Expert Opin Ther Pat, 19: 847-66, pour revue). A l'heure actuelle, seul le phloroglucinol, synthétisé de manière chimique, est commercialisé comme médicament antispasmodique musculotrope (sous l'appellation Spasfon ® en France). Par ailleurs, des mélanges de produits sont aussi commercialisés comme des extraits d'*Ascophyllum nodosum* par la société Algues & Mer d'Ouessant (29, France), des produits comme le Seanol™, extrait d'*Ecklonia cava* ou encore HealSea, extrait de *Fucus vesiculosus* par Diana Naturals (35, France) et quelques autres entreprises dans le monde.

L'un des enjeux majeurs actuels est donc de fournir des méthodes de production de phloroglucinol ou de ses dérivés qui soient efficaces et rapides, et qui permettent d'obtenir ces composés dans des proportions importantes. L'une des possibilités consiste à le produire via une voie de biosynthèse mettant en oeuvre l'utilisation d'enzymes.

Parmi ces enzymes potentiellement capables de synthétiser du phloroglucinol, on distingue notamment les polyketide synthases de type III (PKS III) qui sont des enzymes clés du métabolisme secondaire chez les plantes terrestres (voie des flavonoïdes, biosynthèse de phytoalexines) mais aussi chez les bactéries, les champignons et quelques protozoaires. Les PKS III correspondantes (chalcone synthases, stilbène synthases, pyrone synthases, ...) partagent le même mécanisme réactionnel mais diffèrent par leur spécificité de substrat pour les molécules « starters » et par la stéréochimie de la réaction de cyclisation donnant naissance à un large panel de métabolites secondaires polyphénoliques ayant des fonctions très diverses.

De nombreuses polyketides synthases ont ainsi été isolées à partir de divers organismes, tels que l'algue brune *Sargassum binderi,* les plantes terrestres *Aloe arborescens* et *Arabidopsis thaliana,* l'amibe *Dictyostelium discoideum,* ou encore les bactéries *Mycobacterium tuberculosis, Streptomyces coelicolor* et *Pseudomonas fluorescens* (Baharum et al., Mar Biotechnol, 2011, 13(5) : 845-856 ; Wanibuchi et al., Bioorg Med Chem Lett, 2011, 21 : 2083-2086; Abe et al., J Am Chem Soc, 2005, 127(5) :1362-1363 ; Mizuuchi et al., Biol Pharm Bull, 2008, 31(12) : 2205-2210 ; Austin et al., Nat Chem Biol, 2006, 2(9) : 494-502 ; Sankaranarayanan et al., Nat Struct Mol Biol, 2004, 11(9) : 894-900 ; Izumikawa et al., J Ind Microbiol Biotechnol, 2003, 30 : 510-515 ; WO2006/044290). Parmi ces polyketides synthases, seule la polyketide synthase *PhlD* issue de la bactérie *Pseudomonas fluorescens* est capable de synthétiser du phloroglucinol en utilisant le malonyl-CoA comme molécule « starter » (WO2006/044290). Les autres polyketides synthases, bien qu'utilisant également le malonyl-CoA en tant que molécule « starter », sont malheureusement incapables de synthétiser le phloroglucinol.

Les inventeurs ont identifié une nouvelle polyketide synthase chez l'algue brune marine *Ectocarpus siliculosus,* qui a été appelée PKS1. Pour la première fois, les inventeurs ont produit une polyketide synthase de type III (PKS III) d'algue brune marine de façon recombinante et active dans un système hétérologue (la bactérie *Escherichia coli*) avec un protocole établi de purification à homogénéité permettant de produire environ 5 à 10 mg de protéine pure par litre de culture. Les inventeurs ont également montré que cette PKS1 était capable de synthétiser *in vitro* du phloroglucinol à partir de malonyl-coenzyme A (malonylCoA). De plus, d'autres produits obtenus à partir de malonylCoA et de plusieurs acylCoA à plus ou moins longues chaînes aliphatiques (hexanoylCoA, decanoylCoA, lauroylCoA et palmitoylCoA) sont présents dans les réactions.

L'invention est telle que définie dans l'objet des revendications 1 à 14.

### Acide nucléique, protéine et méthode de production de l'enzyme

La présente invention concerne un acide nucléique isolé consistant en la séquence SEQ ID NO : 2 ou SEQ ID NO : 4, ou en une séquence au moins 85% identique à la séquence SEQ ID NO : 2 ou au moins 95% identique à la séquence SEQ ID NO : 4, caractérisé en ce qu'il code pour une polyketide synthase de type III (PKSIII), ou en une séquence complémentaire de SEQ ID NO : 2 ou de SEQ ID NO : 4.

De manière préférée, ledit acide nucléique isolé code pour une polyketide synthase de type III.

La présente invention concerne en outre un acide nucléique isolé codant pour une polyketide synthase de type III (PKSIII) consistant en :
a) la séquence nucléotidique SEQ ID NO : 2,
b) la séquence nucléotidique SEQ ID NO : 4,
c) la séquence complémentaire de SEQ ID NO : 2 ou de SEQ ID NO : 4,
d) une séquence au moins 85% identique à SEQ ID NO : 2 ou au moins 95% identique à la séquence à SEQ ID NO : 4,
e) une séquence différant des séquences a) à d) par dégénérescence du code,
f) une séquence nucléotidique s'hybridant en conditions de stringence spécifiques avec au moins l'une des séquences a) à e).

Par « isolé », on entend un composé qui a été isolé d'un organisme vivant, tel qu'une algue, ou un animal, et/ou d'une bibliothèque de composés. De manière préférée, l'acide nucléique isolé provient d'une algue brune marine. De manière encore plus préférée, l'acide nucléique isolé provient de l'algue brune marine *Ectocarpus siliculosus* (*E. siliculosus*).

Préférentiellement, les acides nucléiques selon l'invention sont des acides nucléiques recombinants. Par « acide nucléique recombinant », on entend un acide nucléique, *i.e.* une molécule d'ADN ou d'ARN, qui a subi une manipulation biologique moléculaire.

Par « acide nucléique », on entend la forme polymérique ester phosphate des ribonucléosides (adénosine, guanosine, uridine ou cytidine ; "molécules d'ARN") ou des désoxyribonucléosides (désoxyadénosine, désoxyguanosine, désoxythymidine ou désoxycytidine ; "molécules d'ADN") sous forme monocaténaire ou sous forme d'une hélice bicaténaire. Des hélices bicaténaires ADN-ADN, ADN-ARN et ARN-ARN sont possibles. Le terme acide nucléique, et en particulier molécule d'ADN ou d'ARN, ne se réfère qu'à la structure primaire ou secondaire de la molécule, et ne se limite aucunement à des formes tertiaires particulières. Ainsi, ce terme comprend l'ADN bicaténaire que l'on trouve, entre autres, dans les molécules d'ADN linéaire ou circulaire (par exemple, fragments de restriction), les virus, les plasmides et les chromosomes. Lorsque l'on évoque la structure de molécules d'ADN bicaténaire particulières, les séquences peuvent être décrites ici conformément à la convention normale qui ne donne la séquence que dans la direction 5' vers 3' le long du brin non transcrit de l'ADN (c'est-à-dire le brin ayant une séquence homologue à l'ARNm).

Par séquence au moins 85% identique à une séquence de référence, on entend que la séquence est identique à la séquence de référence, sauf que la séquence peut inclure jusqu'à quinze altérations de nucléotides tous les 100 nucléotides de la séquence de référence. En d'autres termes, pour obtenir une séquence au moins 85% identique à la séquence de référence, jusqu'à 15% des nucléotides de la séquence peuvent être insérés, délétés, ou substitués par un autre nucléotide.

Le pourcentage d'identité peut être calculé en réalisant un alignement global par paire basé sur l'algorithme d'alignement Needleman-Wunsch pour trouver l'alignement optimal (incluant des "trous" ou "gaps") entre deux séquences sur toute leur longueur, par exemple en utilisant Needle, et en utilisant la matrice BLOSUM62 avec une pénalité d'insertion de « gaps » de 10 et une pénalité d'extension de « gaps » de 0.5.

De manière préférée, la séquence nucléotidique de l'invention, notamment la séquence c) est au moins 90%, 91%, 92%, 93%, 94%, 95%, ou 99% identique à la séquence SEQ ID NO : 2 ou SEQ ID NO : 4.

Par « séquence différant des séquences a) à c) par dégénérescence du code », on entend une séquence qui diffère de la séquence de référence par une substitution conservative comme indiqué dans le tableau ci-dessous.

| **Substitutions conservatives** | **Type d'acide aminé** |
|---|---|
| Ala, Val, Leu, Ile, Met, Pro, Phe, Trp | Acides aminés avec chaînes latérales hydrophobes aliphatiques |
| Ser, Tyr, Asn, Gln, Cys | Acides aminés avec chaînes latérales polaires non chargées |
| Asp, Glu | Acides aminés avec chaînes latérales acides |
| Lys, Arg, His | Acides aminés avec chaînes latérales basiqueq |
| Gly | Chaîne latérale neutre |

Une molécule d'acide nucléique "peut s'hybrider" à une autre molécule d'acide nucléique tel qu'un ADNc, un ADN génomique ou un ARN, lorsque la forme monocaténaire de la molécule d'acide nucléique peut s'hybrider à l'autre molécule d'acide nucléique dans des conditions appropriées de température et de force ionique de la solution (voir Sambrook *et al*., *supra*). Les conditions de température et de force ionique déterminent la "stringence" de l'hybridation. Des conditions d'hybridation de faible stringence, correspondent à un Tₘ de 55 °C, par exemple SSC 5X, SDS 0,1 %, lait 0,25 %, et pas de formamide ; ou formamide 30 %, SSC 5 x, SDS 0,5 %). Des conditions d'hybridation de stringence modérée correspondent à un Tₘ plus élevé (environ 60 °C), par exemple formamide 40 %, avec du SSC 5x ou 6x. Des conditions d'hybridation de stringence élevée correspondent au Tₘ le plus élevé (supérieur ou égale à environ 65 °C), par exemple formamide 50 %, SSC 5x ou 6x. L'hybridation nécessite que les deux molécules d'acide nucléique contiennent des séquences complémentaires, bien qu'en fonction de la stringence de l'hybridation, des mésappariements entre les bases soient possibles. La stringence appropriée à l'hybridation de molécules d'acide nucléique dépend de la longueur des molécules d'acide nucléique et du degré de complémentation, des variables bien connues des hommes du métier. Plus le degré de similarité ou d'homologie entre deux séquences nucléotidiques est élevé, plus la valeur des Tₘ pour les hybrides des molécules d'acide nucléique ayant ces séquences est élevée. La stabilité relative (correspondant à un Tₘ plus élevé) d'hybridations d'acide nucléique décroît dans l'ordre suivant : ARN:ARN, ADN:ARN, ADN:ADN. Pour des hybrides d'une longueur supérieure à 100 nucléotides, les équations pour calculer le Tₘ ont été dérivées (voir Sambrook *et al*., *supra,* 9.50-0.51). Pour l'hybridation avec des molécules d'acide nucléique plus courtes, c'est-à-dire des oligonucléotides, la position des mésappariements devient plus importante, et la longueur de l'oligonucléotide détermine sa spécificité (voir Sambrook *et al*., *supra,* 11.7-11.8). De préférence, une longueur minimale pour une molécule d'acide nucléique pouvant s'hybrider est d'au moins environ 10 nucléotides ; de préférence, d'au moins environ 10 nucléotides ; et plus préférablement, la longueur est d'au moins environ 50 nucléotides ; encore plus préférablement d'au moins 100 nucléotides ; encore plus préférablement d'au moins 1000 nucléotides.

Le terme "conditions d'hybridation spécifique" désigne un Tₘ de 55 °C et utilise des conditions décrites ci-dessus. Dans un mode de réalisation préféré, le Tₘ est égal à 60 °C, dans un mode de réalisation encore plus préféré, le Tₘ est égal à 65 °C.

Une "séquence codante" d'ADN est une séquence d'ADN bicaténaire qui est transcrite et traduite en un polypeptide *in vivo* lorsqu'elle est placée sous le contrôle de séquences régulatrices appropriées. Les frontières de la séquence codante sont déterminées par un codon de début à l'extrémité 5' (amino) et par un codon d'arrêt de la traduction à l'extrémité 3' (carboxy). Une séquence codante peut inclure, sans s'y limiter, des séquences procaryotes, de l'ADNc issu d'ARNm eucaryote, des séquences d'ADN génomique provenant d'ADN eucaryote (par exemple de mammifères), et même des séquences d'ADN synthétiques. Si la séquence codante est destinée à une expression dans une cellule eucaryote, un signal de polyadénylation et une séquence de terminaison de la transcription seront généralement situés en 3' de la séquence codante.

Des séquences de contrôle de la transcription et de la traduction sont des séquences régulatrices d'ADN, telles que des promoteurs, des activateurs, des terminateurs et d'autres séquences similaires, qui permettent l'expression d'une séquence codante dans une cellule hôte. Dans les cellules eucaryotes, les signaux de polyadénylation sont des séquences de contrôle.

Une "séquence promoteur" est une région régulatrice d'ADN capable de lier l'ARN polymérase dans une cellule et d'initier la transcription d'une séquence codante vers l'aval (direction 3'). Dans les objectifs de définition de la présente invention, la séquence promoteur est liée à son extrémité 3' par le site d'initiation de la transcription et s'étend en amont (direction 5') en incluant le nombre minimal de bases ou d'éléments nécessaires pour initier la transcription à des niveaux détectables par rapport au bruit de fond. Dans la séquence promoteur, se trouve un site d'initiation de la transcription (commodément défini, par exemple, par cartographie avec la nucléase S1), ainsi que des domaines de liaison aux protéines (séquences consensus) responsables de la liaison de l'ARN polymérase. Des promoteurs eucaryotes contiendront souvent, mais pas toujours des boîtes "TATA" et des boîtes "CAT".

Une séquence codante est "sous le contrôle" de séquences de contrôle de la transcription et de la traduction dans une cellule, lorsque l'ARN polymérase transcrit la séquence codante en ARNm qui est ensuite traduite en protéine codée par la séquence codante.

Une "séquence signal" peut être incluse avant la séquence codante. Cette séquence code un peptide signal, en position N-terminale de la protéine, qui commande à la cellule hôte de transporter la protéine sur la surface cellulaire ou de sécréter la protéine dans le milieu, et ce peptide signal est généralement sélectivement dégradé par la cellule après exportation. Les séquences signal peuvent se trouver associées avec diverses protéines natives des procaryotes et des eucaryotes. De manière préférée, la séquence signal consiste en ou comprend la séquence 5'-ATGTCTTCTGCTGCGGTTGCTATGCTGGCTGACCCGACTGTCCAGATCGCTCTGGCGTGCCTGGTGGTGTCTCTCTTCGTTGTGCTGCAGTCGGTCAAAAAG-3' (SEQ ID NO :5). De manière préférée, la séquence signal code pour le peptide signal de séquence MSSAAVAMLADPTVQIALACLVVSLFVVLQSVKK (SEQ ID NO : 6).

Une « séquence étiquette » (ou « Tag ») peut également être incluse avant ou après la séquence codante. Cette séquence code généralement pour une répétition d'histidine, en position N-terminale de la protéine, et permet la purification de la protéine. De manière préférée, la séquence étiquette est placée avant la séquence codante, et code pour la séquence de six histidines SEQ ID NO : 7 (HHHHHH), la séquence étiquette peut donc avoir pour séquence nucléotidique la séquence SEQ ID NO : 8 (5'-CAYCAYCAYCAYCAYCAY-3'). De manière encore plus préférée, la séquence étiquette a pour séquence d'acides aminés la séquence MRGSHHHHHHGS (SEQ ID NO : 9). La séquence nucléotidique correspondant à la séquence étiquette codant pour la séquence SEQ ID NO : 9 peut être la séquence 5'-ATGCGCGGCAGCCATCATCATCATCATCATGGCAGC-3' (SEQ ID NO : 10).

Dans un mode de réalisation particulièrement préféré, l'acide nucléique selon l'invention code pour une PKSIII capable de synthétiser du phloroglucinol à partir de malonyl-CoA seul ou en combinaison avec d'autres substrats, par exemple l'acétyl-CoA, l'hexanoyl-CoA, le décanoyl-CoA, le lauroyl-CoA, ou le palmitoyl-CoA.

L'invention concerne également une protéine codée par un acide nucléique isolé selon l'invention. De manière préférée, ladite protéine est codée par un acide nucléique consistant en la séquence SEQ ID NO : 2 ou SEQ ID NO : 4. De manière encore plus préférée, ladite protéine consiste en la séquence SEQ ID NO : 1 ou SEQ ID NO : 3. Alternativement, ladite protéine consiste en une séquence ayant au moins 93% d'identité, préférentiellement au moins 95% d'identité, ou encore plus préférentiellement 99% d'identité avec la séquence SEQ ID NO : 1 ou SEQ ID NO : 3.

De préférence, ladite protéine est une PKSIII capable de synthétiser du phloroglucinol à partir de malonyl-CoA seul ou en combinaison avec d'autres substrats, par exemple l'acétyl-CoA, l'hexanoyl-CoA, le décanoyl-CoA, le lauroyl-CoA, ou le palmitoyl-CoA.

Par séquence au moins 93% identique à une séquence de référence, on entend que la séquence est identique à la séquence de référence, sauf que la séquence peut inclure jusqu'à sept altérations d'acides aminés tous les 100 acides aminés de la séquence de référence. En d'autres termes, pour obtenir une séquence au moins 93% identique à la séquence de référence, jusqu'à 7% des acides aminés de la séquence peuvent être insérés, délétés, ou substitués par un autre acide aminé. De préférence, la séquence au moins 93% identique est une séquence homologue à la séquence de référence, c'est-à-dire qu'elle diffère de la séquence de référence par une ou plusieurs substitutions conservatives.

Le pourcentage d'identité peut être calculé en réalisant un alignement global par paire basé sur l'algorithme d'alignement Needleman-Wunsch pour trouver l'alignement optimal (incluant des "trous" ou "gaps") entre deux séquences sur toute leur longueur, par exemple en utilisant Needle, et en utilisant la matrice BLOSUM62 avec une pénalité d'insertion de « gaps » de 10 et une pénalité d'extension de « gaps » de 0.5.

Par « substitutions conservatives », on entend le remplacement d'un acide aminé par un autre, sans altérer la conformation et la fonction de la protéine, incluant, mais sans être limitant, le remplacement d'un acide aminé par un autre acide aminé ayant les mêmes propriétés (telles que par exemple, les mêmes polarités, potentiels de liaison à l'hydrogène, propriétés acide, basique, hydrophobe, aromatique, et autres). Les acides aminés ayant les mêmes propriétés sont connus de l'homme du métier. Par exemple, l'arginine, l'histidine et la lysine sont des acides aminés basiques hydrophiles qui peuvent être interchangeables. De manière similaire, l'isoleucine, un acide aminé hydrophile, peut être remplacé par la leucine, la méthionine ou la valine. Les acides aminés neutres hydrophiles, qui peuvent être substitués entre eux, incluent l'asparagine, la glutamine, la sérine et la thréonine.

Par « substitué » ou « modifié », la présente invention inclut les acides aminés qui ont été altérés ou modifiés à partir d'acides aminés naturels.

Ainsi, il doit être compris que dans le contexte de l'invention, une substitution conservative est reconnue dans l'état de l'art comme une substitution d'un acide aminé par un autre acide aminé ayant les mêmes propriétés.

Des exemples de substitutions conservatives sont donnés dans le Tableau 1 ci-dessous :

**Tableau 1 : Substitutions conservatives I**

| Caractéristiques de la chaîne latérale | Acide aminé |
|---|---|
| Non polaire | G A P I L V |
| Polaire non chargée | C S T M N Q |
| Polaire chargée | D E K R |
| Aromatique | H F W Y |
| Autres | N Q D E |

Alternativement, les acides aminés peuvent être groupés comme décrit par Lehninger (1975, Biochemistry, Second Edition, Worth Publishers, Inc. New-York: NY., pp. 71-77), comme décrit dans le Tableau 2 ci-dessous :

**Tableau 2 : Substitutions conservatives II**

| Caractéristiques de la chaîne latérale | | Acide aminé |
|---|---|---|
| Non polaire | Aliphatique | A L I V P |
| | Aromatique | F W |
| | Contenant du Soufre | M |
| | Cas à part | G |
| Polaire non chargée | Hydroxyle | S T Y |
| | Amides | N Q |
| | Sulfhydryle | C |
| | Cas à part | G |
| Chargée positivement (basique) | | K R H |
| Chargée négativement (acide) | | D E |

Dans une autre alternative, des exemples de substitutions conservatives sont données dans le Tableau 3 ci-dessous :

**Tableau 3 : Substitutions conservatives III**

| Résidu original | Exemple de substitution |
|---|---|
| A | V L I |
| R | K Q N |
| N | Q H K R |
| D | E |
| C | S |
| G | N |
| E | D |
| H | N Q K R |
| I | L V M A F |
| L | I V M A F |
| K | R Q N |
| M | L F I |
| F | L V I A |
| P | G |
| S | T |
| T | S |
| W | Y |
| Y | W F T S |
| V | I L M F A |

L'invention concerne également un vecteur comprenant un acide nucléique selon l'invention, dans lequel ledit acide nucléique est placé sous le contrôle de signaux (*i.e*. un promoteur, un terminateur et/ou un enhancer) permettant l'expression de l'acide nucléique selon l'invention. Le vecteur peut en outre comprendre un gène de résistance à un antibiotique, tel que l'ampicilline ou la kanamycine.

Le terme « vecteur » désigne un élément extrachomosomique qui peut porter un gène non essentiel pour le métabolisme cellulaire, et qui est généralement un ADN circulaire double brin. L'élément extrachomosomique peut être une séquence auto-réplicative, une séquence de phage ou une séquence nucléotidique, un ADN ou ARN simple ou double brin, un plasmide, un cosmide. Généralement, un vecteur contient des séquences régulatrices de la transcription ou de la traduction, un marqueur de sélection, ou une séquence permettant l'autoréplication ou une insertion chromosomique. Un vecteur approprié inclut la région 5' d'un gène qui régule l'initiation de la transcription *(i.e.* un promoteur) et une région 3' qui contrôle la terminaison de la transcription *(i.e.* un terminateur). Le promoteur peut par exemple être celui de CYC1, HIS3, GAL1, GAL10, ADH1, PGK, PHO5, GAPDH, ADC1, TRP1, URA3, LEU2, ENO, TPI, lac, trp, λP_{L}, λP_{R}, T7, tac, bactériophage T5 ou trc. Le terminateur peut être dérivé de nombreux gènes d'une cellule hôte préférée et peut, optionnellement, être omis. Préférentiellement, le vecteur est un plasmide pQE-80L (Qiagen) contenant le promoteur du bactériophage T5 inductible par l'Isopropyl-β-D-thiogalactopyranoside (IPTG).

Les acides nucléiques et/ou le vecteur selon l'invention peuvent être utilisés pour transformer une cellule ou un organisme hôte, *i.e.* pour l'expression ou la production d'une protéine selon l'invention.

Ainsi, un autre aspect de l'invention concerne un hôte ou une cellule hôte qui contient un acide nucléique ou un vecteur selon l'invention, ou qui exprime (ou qui est capable d'exprimer dans des conditions appropriées) une protéine selon l'invention. Des hôtes ou cellules hôtes adaptés sont connus de l'homme du métier, et peuvent par exemple être n'importe quel champignon, cellule ou lignée cellulaire eucaryote ou procaryote, organismes eucaryotes ou procaryotes. Par exemple, l'hôte ou la cellule hôte peut être (i) une souche bactérienne, incluant, sans être limitant, les lignées de bactéries gram-négatives telles que les lignées *d'Escherichia,* par exemple *Escherichia coli*; de *Proteus,* par exemple *Proteus mirabilis;* de *Pseudomonas,* par exemple *Pseudomonas fluorescens*; et les souches de bactéries gram-positives telles que les lignées de *Bacillus,* par exemple *Bacillus subtilis* ou *Bacillus brevis*; de *Streptomyces,* par exemple *Streptomyces lividans*; de *Staphylococcus,* par exemple de *Staphylococcus carnosus*; et de *Lactococcus,* par exemple *Lactococcus lactis* ; (ii) une cellule fongique, incluant, sans être limitant les cellules des espèces *Trichoderma,* par exemple *Trichoderma reesei*; *Neurospora,* par exemple *Neurospora crassa*; *Sordaria,* par exemple *Sordaria macrospore*; *Aspergillus,* par exemple *Aspergillus niger* ou *Aspergillus sojae;* ou d'autres champignons filamenteux ; (iii) une levure, incluant, sans être limitant, les cellules des espèces *Saccharomyces,* par exemple *Saccharomyces cerevisiae; Schizosaccharomyces,* par exemple *Schizosaccharomyces pombe; Pichia,* par exemple *Pichia pastoris* ou *Pichia methanolica*; *Hansenula,* par exemple *Hansenula polymorpha*; *Kluyveromyces,* par exemple *Kluyveromyces lactis; Arxula,* par exemple *Arxula adeninivorans; Yarrowia,* par exemple *Yarrowia lipolytica*; (iv) une cellule ou lignée cellulaire d'amphibien, telle que les ovocytes de *Xenopus*; (v) une cellule ou lignée cellulaire d'insecte, telle que les lignées SF9 ou Sf21; (vi) une plante ou une cellule de plante, par exemple une plante de tabac ; et/ou (vii) une cellule ou lignée cellulaire de mammifère, incluant, sans être limitant, les cellules CHO, BHK, HeLa, COS (*i.e*. COS-7) et PER.C6. Préférentiellement, la cellule hôte est une bactérie de type *Escherichia coli,* et plus préférentiellement la cellule hôte est la souche *E. coli* BL21-CodinPlus-RILP (Stratagene).

Un autre aspect de l'invention concerne une méthode de production d'une polyketide synthase, comprenant les étapes de :
a) culture d'une cellule hôte selon l'invention, dans des conditions permettant l'expression d'une polyketide synthase recombinante,
b) extraction et/ou purification, de ladite polyketide synthase recombinante.

La méthode comprend en outre une étape de transformation d'une cellule hôte à l'aide d'un vecteur recombinant tel que défini précédemment avant l'étape a). En conséquence, la méthode selon l'invention peut comprendre les étapes de :
a0) transformation d'une cellule hôte à l'aide d'un vecteur tel que défini précédemment ;
a) culture de ladite cellule transformée, dans des conditions permettant l'expression d'une polyketide synthase recombinante,
b) extraction et/ou purification de ladite polyketide synthase recombinante.

La transformation à l'étape a0) peut être réalisée par des procédés connus de l'homme du métier, par exemple, par transfection, électroporation, électrotransfert, microinjection, transduction, fusion de cellules, DEAE-dextran, précipitation au phosphate de calcium, lipofection, utilisation de canon à gènes, ou un transporteur de vecteur d'ADN (voir par exemple, Wu et al., 1992, J. Biol Chem. 267 :963-967 ; Wu et al., 1988, J. Biol Chem. 263 :14621-14624 ; Hartmut *et al*., demande de brevet canadienne No. 2 012 311, publiée le 15 mars 1990). De manière préférée, la transformation à l'étape a0) est réalisée par choc thermique sur des cellules rendues chimiquement compétentes, dans un milieu de culture approprié. De manière préférée, le choc thermique est réalisé entre 35°C et 50°C pendant 30 secondes à 1 min suivi d'un retour dans la glace pendant 1 à 3 min. De manière encore plus préférée, le choc thermique est réalisé à 42°C pendant 45 secondes suivi d'un retour dans la glace pendant 2 min Par milieu de culture approprié, on entend un milieu de culture permettant la croissance des cellules transformées. Un tel milieu de culture est connu de l'homme du métier. Par exemple, il s'agit d'un milieu incluant, sans être limitant, un substrat carboné ou une source de carbone qui peut être métabolisé(e) par la cellule transformée. Le substrat carboné ou la source de carbone peut être sélectionné(e) parmi les monosaccharides, les oligosaccharides, les polysaccharides, les substrats simple-carbone, et un mélange de ces composés. Optionnellement, le milieu de culture contient un antibiotique, tel que l'ampicilline ou la kanamycine.

De manière préférée, la cellule transformée à l'étape a0) est une bactérie de type *E. coli,* telle que la souche *E. coli* BL21-CodinPlus-RILP (Stratagene), transformée avec un vecteur et/ou un acide nucléique selon l'invention, et cultivée en utilisant un milieu adapté, tel que le milieu Luria-Broth (LB), de préférence contenant au moins 100 µg/ml d'ampicilline.

La culture réalisée à l'étape a) a pour but de permettre l'expression de la polyketide synthase recombinante. Elle peut être réalisée par des procédés connus de l'homme du métier, par exemple, à l'aide d'un bioréacteur, aussi appelé fermenteur. Le bioréacteur est une cuve hermétique munie d'un système d'agitation, d'un système d'aération, de sondes servant à mesurer divers paramètres (pH, température, oxygène dissout) et des pores d'alimentation permettant un dosage précis de la composition du milieu de culture en cours de fermentation. La fermentation dans le bioréacteur s'effectue en deux phases : une phase I de croissance durant laquelle les cellules se divisent à un rythme accéléré, puis une phase II d'accumulation ou d'induction menant à l'expression de la protéine recombinante. L'homme du métier est capable de déterminer les conditions de culture appropriées pour permettre la phase I de croissance et la phase II d'accumulation ou d'induction. Dans un mode de réalisation particulier, les phases I et II peuvent être réalisées comme décrit dans l'exemple 1.3.

L'extraction et/ou la purification à l'étape b) peuvent être réalisées à l'aide de procédés connus de l'homme du métier. A titre d'exemple non limitatif, l'extraction peut être réalisée par lyse des cellules à l'aide d'une presse de French, par sonication, ou par voie enzymatique, ou par toute autre technique standard. La lyse des cellules peut être suivie d'une étape de filtration, et/ou centrifugation du lysat. A titre d'exemple non limitatif, la purification peut être réalisée à l'aide d'une chromatographie (sur colonne échangeuse d'ions, d'affinité, ou de séparation par taille), d'une centrifugation, d'une solubilité différentielle ou par toute autre technique standard. Dans un mode de réalisation particulier, l'extraction et/ou la purification peuvent être réalisées comme décrit dans l'exemple 1.3.

De préférence, ladite méthode de production permet de produire au moins 1 mg/L à 20 mg/L de protéine pure, et de préférence encore au moins 5 à 10 mg/L de protéine pure.

### Méthode de production de composés polyphénoliques

Les inventeurs ont ainsi identifié qu'une polykétide synthase de type III (PKSIII) extraite d'une algue brune était capable de synthétiser du phloroglucinol et/ou l'un de ses dérivés à partir de divers substrats carbonés, et plus particulièrement à partir de malonyl-CoA.

La présente invention décrit une méthode de production d'au moins un composé polyphénolique, dans laquelle :
- une polyketide synthase de type III (PKSIII) d'algue brune est mise en contact avec au moins un substrat carboné dans des conditions permettant la production majoritaire d'au moins un composé polyphénolique,
- ledit composé phénolique produit est le phloroglucinol et/ou un de ses dérivés.

La présente invention concerne également une méthode de production d'au moins un composé polyphénolique, dans laquelle :
- une polyketide synthase de type III (PKSIII) d'algue brune est mise en contact avec au moins un substrat carboné dans des conditions permettant la production majoritaire d'au moins un composé polyphénolique, caractérisée en ce que ladite PKSIII comprend ou consiste en :
   - la séquence d'acides aminés SEQ ID NO : 1 ou une séquence ayant au moins 93% d'identité avec SEQ ID NO : 1, ou une séquence susceptible d'être codée par la séquence nucléotidique SEQ ID NO : 2 ou une séquence ayant au moins 85% d'identité avec SEQ ID NO : 2, ou
   - la séquence d'acides aminés SEQ ID NO : 3 ou une séquence ayant au moins 93% d'identité avec SEQ ID NO : 3, ou une séquence susceptible d'être codée par la séquence nucléotidique SEQ ID NO : 4 ou une séquence ayant au moins 85% d'identité avec SEQ ID NO : 4.
- ledit composé phénolique produit est le phloroglucinol ou un de ses dérivés,
- ledit substrat est sélectionné parmi le malonyl-CoA, l'hexanoyl-CoA, le decanoyl-CoA, le lauroyl-CoA et/ou le palmitoyl-CoA.

De préférence, la méthode comporte une étape de récupération du phloroglucinol et/ou d'un de ses dérivés. Cette étape de récupération peut comprendre une étape d'extraction et/ou une étape de purification.

Par « composé polyphénolique », on entend un composé issu du métabolisme secondaire du règne végétal, *i.e.* plantes terrestres et macrophytes aquatiques, qui présente au moins un cycle aromatique à 6 carbones (phénol) lui-même porteur d'une ou plusieurs fonctions hydroxyles (OH). On distingue plusieurs familles de molécules dont la structure est relativement proche : les flavonoïdes (pigments végétaux jaunes-orangés), les anthocyanes (composés de couleurs rouge à violet responsable de la couleur pourpre des raisins rouges) et les tannins.

Par « polyketide synthase de type III », on entend une enzyme capable de synthétiser du phloroglucinol et/ou l'un de ses dérivés à partir d'un substrat carboné. De manière préférée, la PKSIII est obtenue à partir d'une algue brune marine, de manière encore préférée, la PKSIII est obtenue à partir d'une algue brune marine du genre *Ectocarpus,* et de manière encore plus préférée, la PKSIII est issue de l'algue brune marine *Ectocarpus siliculosus.*

Dans un mode de réalisation préféré, la PKSIII est produite par la méthode selon l'invention décrite ci-dessus.

Selon un aspect particulier de l'invention, la PKSIII comprend ou consiste en une protéine selon l'invention. Plus particulièrement la PKSIII comprend ou consiste en la séquence SEQ ID NO : 1 ou SEQ ID NO : 3 ou une séquence ayant au moins 93%, 95% ou 99% d'identité avec la séquence SEQ ID NO : 1 ou SEQ ID NO : 3.

La séquence ayant au moins 93% d'identité avec SEQ ID NO : 1 ou SEQ ID NO : 3 peut différer de la séquence de référence (*i.e.* SEQ ID NO : 1 ou SEQ ID NO : 3) par une ou plusieurs substitution(s) conservatives(s). Les termes « substitutions conservatives » sont tels que définis ci-dessus.

Selon un aspect particulièrement préféré, la PKSIII consiste en la séquence SEQ ID NO : 1 ou SEQ ID NO : 3.

Selon un autre aspect particulier de l'invention, ladite la PKSIII comprend ou consiste en une séquence susceptible d'être codée par la séquence nucléotidique SEQ ID NO : 2 ou une séquence ayant au moins 85%, 90%, 91%, 92%, 93%, 94%, 95%, ou 99% d'identité avec SEQ ID NO : 2.

Selon un autre aspect particulier de l'invention, ladite la PKSIII comprend ou consiste en une séquence susceptible d'être codée par la séquence nucléotidique SEQ ID NO : 4 ou une séquence ayant au moins 85%, 90%, 91%, 92%, 93%, 94%, 95%, ou 99% d'identité avec SEQ ID NO : 4.

La séquence ayant au moins 85% d'identité avec SEQ ID NO : 2 ou SEQ ID NO : 4 peut différer de la séquence de référence *(i.e.* SEQ ID NO : 2 ou SEQ ID NO : 4) par une ou plusieurs substitution(s) conservatives(s). Les termes « substitutions conservatives » sont tels que définis ci-dessus.

Par « substrat carboné », on entend un composé utilisé en tant que source de carbone. Par exemple, le substrat carboné inclut des composés de type métabolites, tels que les chaînes carbonées C1 à C18, les acides gras, les mono-, di-, tri-glycérides, les polyols, les phospholipides, les phosphoacides, les monosaccharides, les acides aminés, les nucléotides, les homo- ou hétéro-oligomères ou polymères hydrolysables de ces composés, et les formes biologiquement actives de ces composés. Lesdits métabolites peuvent être de n'importe quelle origine, biologique ou synthétique. D'autres exemples de composés sont les composés aromatiques, aliphatiques et cycloaliphatiques C1-C18.

Selon l'invention, le substrat carboné est choisi parmi le malonyl-CoA, l'acétyl-CoA, l'hexanoyl-CoA, le décanoyl-CoA, le lauroyl-CoA, et/ou le palmitoyl-CoA.

Dans un mode de réalisation particulièrement préféré, le substrat carboné est le malonyl-CoA.

Par « phloroglucinol », on entend le benzène-1,3,5-triol, son numéro CAS est le 108-73-6.

Par « dérivé du phloroglucinol », on entend notamment les composés suivants : l'acétyl-phloroglucinol, le lauroyl-phloroglucinol, le palmitoyl-phloroglucinol, l'hexanoyl-phloroglucinol, le décanoyl-phloroglucinol.

Dans un aspect particulièrement préféré, le substrat carboné est le malonyl-CoA et le composé phénolique produit consiste en ou comprend le phloroglucinol.

Dans un autre aspect particulièrement préféré, le substrat carboné est le malonyl-CoA et l'acétyl-CoA, et le composé phénolique produit consiste en ou comprend le phloroglucinol et l'acétyl-phloroglucinol.

Dans un autre aspect particulièrement préféré, le substrat carboné est le malonyl-CoA et le lauroyl-CoA et le composé phénolique produit consiste en ou comprend le phloroglucinol et le lauroyl-phloroglucinol.

Dans un autre aspect particulièrement préféré, le substrat carboné est le malonyl-CoA et le palmitoyl-CoA et le composé phénolique produit consiste en ou comprend le phloroglucinol et le palmitoyl-phloroglucinol.

Dans un autre aspect particulièrement préféré, le substrat carboné est le malonyl-CoA et l'hexanoyl-CoA et le composé phénolique produit consiste en ou comprend le phloroglucinol et l'hexanoyl-phloroglucinol.

Dans un autre aspect particulièrement préféré, le substrat carboné est le malonyl-CoA et le décanoyl-CoA et le composé phénolique produit consiste en ou comprend le phloroglucinol et le décanoyl-phloroglucinol.

Par production majoritaire de phloroglucinol ou de l'un de ses dérivés, on entend que le phloroglucinol ou l'un de ses dérivés est le produit qui est retrouvé en concentration la plus importante par rapport aux autres composés produits. Préférentiellement, la production de phloroglucinol ou de l'un de ses dérivés est d'au moins 30%, 40%, 50%, 60%, 70%, 80%, 90% ou 100%.

L'étape de mise en contact de la polyketide synthase avec au moins un substrat carboné est connue de l'homme du métier. De manière préférée, mais non limitante, cette étape est réalisée comme décrit dans les exemples 1.4 ou 1.5. Plus particulièrement, elle comprend les étapes de (i) mise en contact de la polyketide synthase avec la source carbonée dans un milieu approprié, (ii) incubation du mélange ainsi obtenu.

Le milieu approprié à l'étape (i) peut être un milieu HCl-EDTA dont les concentrations respectives sont comprises entre 30 et 70 mM d'HCI et 0,5 et 2 mM d'EDTA, préférentiellement comprises entre 45 et 55 mM d'HCI et 0,8 et 1,2 mM d'EDTA, encore plus préférentiellement les concentrations d'HCI et d'EDTA sont respectivement de 50 mM et 1 mM. De préférence, le pH du milieu est compris entre 6 et 8, et de préférence encore, le pH est de 7,5 environ.

L'incubation (ii) est de préférence réalisée à température ambiante, *i.e.* à une température comprise entre 20°C et 30°C, et pendant une durée comprise entre 30 min et 8h. Encore de préférence, l'incubation est réalisée à une température comprise entre 22°C et 28°C, et de préférence encore l'incubation est réalisée à 25°C environ. De préférence, l'incubation est réalisée pendant une durée comprise entre 1 h et 5h, et de préférence encore l'incubation est réalisée pendant 1h, 2h, 3h, 4h ou 5h environ. Dans un mode de réalisation particulièrement préférée, l'incubation est réalisée à 25°C environ pendant 1h, 2h, 3h, 4h ou 5h environ.

L'étape d'extraction et/ou de purification peut être réalisée par des techniques connues de l'homme du métier. Par exemple, l'extraction peut être réalisée à l'aide d'acétate d'éthyle. L'étape de purification peut, par exemple, être réalisée par chromatographie sur couche mince (CCM) ou par chromatographie en phase gazeuse couplée à la spectrométrie de masse (GC-MS).

Les composés polyphénoliques produits par la méthode selon l'invention, tels que le phloroglucinol et ses dérivés, peuvent être utilisés dans de nombreuses applications dans les domaines pharmaceutique, nutraceutique, cosmétique, agroalimentaire ou phytosanitaire.

Ainsi, l'invention décrit également une méthode de production d'un composé pharmaceutique, nutraceutique, cosmétique, agroalimentaire ou phytosanitaire, ladite méthode comprenant les étapes de :
a) production d'au moins un composé polyphénolique par la méthode selon l'invention ;
b) optionnellement, modification dudit composé phénolique obtenu à l'étape a) pour produire un composé pharmaceutique, nutraceutique, cosmétique, agroalimentaire ou phytosanitaire,
c) addition d'un support, véhicule, excipient, diluant et/ou adjuvant acceptable pour l'application retenue.

L'invention décrit également l'utilisation des composés polyphénoliques produits par la méthode selon l'invention pour la préparation de compositions pharmaceutiques, nutraceutique, cosmétique, agroalimentaire ou phytosanitaire.

L'invention décrit aussi ces compositions.

Plus particulièrement, les compositions pharmaceutiques ou nutraceutiques peuvent contenir des supports ou similaires (véhicules, excipients, diluants) et/ou des adjuvants pharmaceutiquement acceptables. Utilisé ici, le terme "pharmaceutiquement acceptable" signifie de préférence approuvé par une agence réglementaire gouvernementale, en particulier recommandée dans la Pharmacopée américaine ou européenne, pour une utilisation chez les animaux, et plus particulièrement les humains. Des supports pharmaceutiques ou similaires appropriés sont notamment décrits dans "Remington's Pharmaceutical Sciences" par E.W. Martin.

Ces supports ou similaires pharmaceutiquement acceptables peuvent être des liquides stériles, tels que l'eau et des huiles, incluant les liquides issus du pétrole, des animaux, des végétaux, ou d'origine synthétique, tel que l'huile d'arachide, l'huile de soja, l'huile minérale, l'huile de sésame, et d'autres huiles similaires. L'eau est un support préféré lorsque la composition pharmaceutique est administrée par voie intraveineuse. Des solutions salines et des solutions aqueuses de dextrose et de glycérol peuvent également être employées comme supports liquides, en particulier pour les solutions injectables. Des excipients pharmaceutiques appropriés comprennent le mannitol, la sérumalbumine humaine (HSA), l'amidon, le glucose, le lactose, le saccharose, la gélatine, le malt, le riz, une farine, la craie, le gel de silice, le carbonate de magnésium, le stéarate de magnésium, le stéarate de sodium, le monostéarate de glycérol, le talc, le chlorure de sodium, le lait écrémé en poudre, le glycérol, le propylène, le glycol, l'eau, l'éthanol et d'autres excipients similaires. Ces compositions peuvent prendre la forme de solutions, de suspensions, de comprimés, de pilules, de capsules, de poudres, de formulations à libération prolongée et d'autres formes similaires.

Ces compositions contiendront une quantité diagnostique ou thérapeutique efficace du composé actif avec une quantité appropriée du support ou similaire de façon à fournir la forme pour une administration appropriée au patient. Bien que l'injection intraveineuse soit une forme très efficace d'administration, d'autres modes peuvent être employés, tels qu'une injection, ou une administration orale, nasale ou parentérale.

Plus particulièrement, les compositions cosmétiques peuvent contenir un véhicule cosmétiquement acceptable. Par " véhicule cosmétiquement acceptable ", on entend un véhicule adapté pour une utilisation en contact avec des cellules humaines et animales, en particulier les cellules de l'épiderme, sans toxicité, irritation, réponse allergique indue et similaire, et proportionné à un rapport avantage/risque raisonnable. Comme exemple de véhicule cosmétiquement acceptable, on peut citer notamment l'eau, en particulier l'eau distillée.

Les compositions cosmétiques décrites peuvent comprendre en outre tout additif usuellement utilisé dans le domaine cosmétique, tel que des séquestrants, des antioxydants, des conservateurs, des charges, des électrolytes, des humectants, des colorants, des bases ou des acides usuels, minéraux ou organiques, des parfums, des huiles essentielles, des actifs cosmétiques, des hydratants, des vitamines, des acides gras essentiels, des sphingolipides, des composés autobronzants, des agents apaisants et protecteurs de la peau. Bien entendu, l'homme du métier veillera à choisir ce ou ces éventuels composés complémentaires et/ou leur quantité, de manière telle que les propriétés avantageuses de la composition décrite ne soient pas, ou substantiellement pas, altérées. Ces additifs peuvent être présents dans la composition à raison de 0,001 % à 20 % en poids par rapport au poids total de la composition.

De façon connue, les compositions cosmétiques décrites peuvent contenir des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique et, par exemple, de 0,01 % à 20 % du poids total de la composition. Ces adjuvants, selon leur nature, peuvent être introduits dans la phase grasse, dans la phase aqueuse et/ou dans les vésicules lipidiques.

L'application d'une composition cosmétique est effectuée par voie topique sur la peau, y compris les muqueuses, notamment les lèvres ou les phanères. L'application d'une composition cosmétique peut également être effectuée par injection intradermique.

Les compositions cosmétiques décrites peuvent se présenter sous toutes les formes galéniques normalement utilisées pour une application topique, sous forme d'onguent, de crème, d'huile, de lait, de pommade, de poudre, de tampon imbibé, de solution, de gel, de spray, de lotion, de suspension, de savon.

Les compositions décrites peuvent être des compositions cosmétiques sous la forme d'émulsion huile-dans-eau ou eau-dans-huile, ou d'émulsion multiple, de microémulsion, de gel hydroalcoolique, de crème, d'huile, de lotion hydroalcoolique.

Les compositions cosmétiques décrites sont particulièrement utiles, pour hydrater, apaiser, réparer et/ou protéger la peau.

Ces compositions cosmétiques sont également particulièrement avantageuses pour lutter contre le vieillissement cutané, à savoir notamment les phénomènes de rides, de perte de tonicité et d'élasticité dus aux modifications structurales de la peau en raison de l'âge.

Les compositions cosmétiques décrites sont également utiles pour protéger la peau des agressions extérieures, telles que notamment les rayons ultraviolets ou les pollutions de l'air.

Les compositions phytosanitaires décrites peuvent comprendre en outre un ou plusieurs tensioactifs, conservateurs, dispersants, mouillants, émulsionnants, anti-mousse, de l'eau.

Les compositions phytosanitaires décrites peuvent être formulées sous différentes formes, par exemple sous la forme de poudres mouillables, granulés dispersibles, suspensions concentrées, poudres pour poudrage.

L'invention décrit également l'utilisation du phloroglucinol et/ou un ou plusieurs de ses dérivés produit(s) par la méthode selon l'invention pour le traitement de désordres spasmodiques, maladies virales, maladies parasitaires, maladies microbiennes, maladies fongiques, désordres dermatologiques, l'hypertension, l'ostéoporose, maladies inflammatoires, maladies vasculaires, désordres sexuels, cancers, diabète, maladies neurodégénératives, la dépression, et l'allergie.

L'invention décrit également des méthodes de traitement de désordres spasmodiques, maladies virales, maladies parasitaires, maladies microbiennes, maladies fongiques, désordres dermatologiques, l'hypertension, l'ostéoporose, maladies inflammatoires, maladies vasculaires, désordres sexuels, cancers, diabète, maladies neurodégénératives, la dépression, et l'allergie, comprenant l'administration de phloroglucinol et/ou d'un ou plusieurs de ses dérivés à un patient le nécessitant.

Les désordres spasmodiques peuvent notamment comprendre les troubles fonctionnels du tube digestif (colites) et des voies biliaires, les coliques néphrétiques ou hépatiques, les douleurs gynécologiques, et les contractions au cours de la grossesse.

Les maladies virales peuvent notamment comprendre les infections au virus de l'immunodéficience acquise (VIH) et au virus de l'herpès.

Les maladies parasitaires peuvent notamment comprendre la malaria.

Les maladies microbiennes peuvent notamment comprendre les infections dues à des bactéries Gram-négatives ou Gram-positives, et plus particulièrement les bactéries de types *Streptococcus mutans, Porphyromonas gingivalis, Bacillus subtilis* et *Staphylococcus aureus.*

Les maladies fongiques peuvent notamment comprendre les infections dues à *Aspergillus niger, Aspergillus flavus, Mucor* et *Cladosporium.*

Les désordres dermatologiques peuvent notamment comprendre le psoriasis, l'alopécie de la peau, les problèmes de cicatrisation de la peau, et le vieillissement cutané à savoir notamment les phénomènes de rides, de perte de tonicité et d'élasticité dus aux modifications structurales de la peau en raison de l'âge.

Les maladies vasculaires peuvent notamment comprendre la maladie de Raynaud et l'acrocyanose.

Les maladies neurogénératives peuvent notamment comprendre la maladie d'Alzheimer, le syndrome de Kiloh Nevin, le syndrome du canal carpien, la paralysie de Tardy Ulnar, le syndrome du canal de Guyon.

Les désordres sexuels peuvent notamment comprendre des troubles de l'érection.

Le phloroglucinol ou ses dérivés peuvent être utilisés seuls ou en combinaison avec d'autres composés ayant une activité thérapeutique pour le traitement des désordres cités ci-dessus.

L'invention va être expliquée plus en détail par les exemples suivants, sans en limiter sa portée.

### DESCRIPTION DES FIGURES

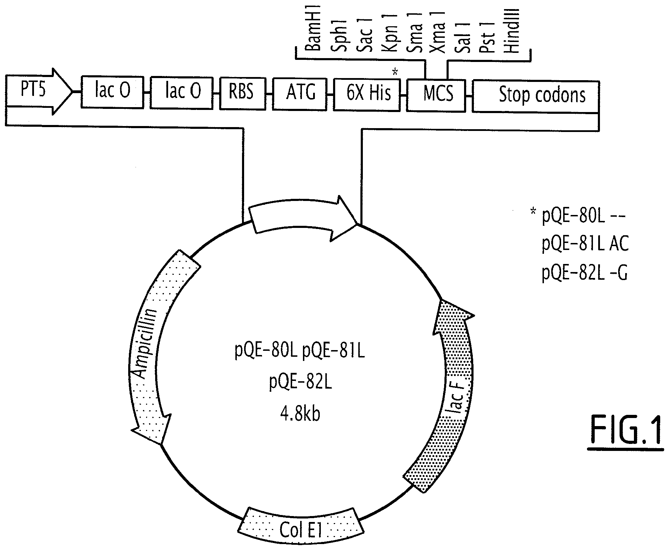

**Figure 1****.** La figure 1 représente le vecteur d'expression pQE-80L (Qiagen) contenant le promoteur du bactériophage T5 inductible par l'Isopropyl-b-D-thiogalactopyranoside (IPTG) utilisé pour l'expression de la protéine PKS1.

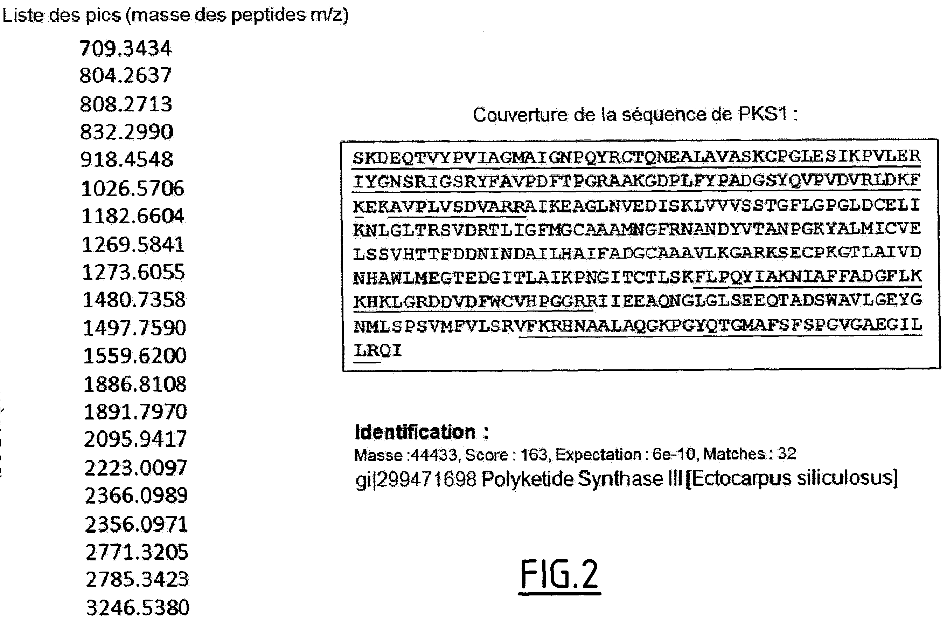

**Figure 2****.** La figure 2 représente l'analyse en spectrométrie de masse de la protéine PKS1 recombinante purifiée d'*E*. *siliculosus.*

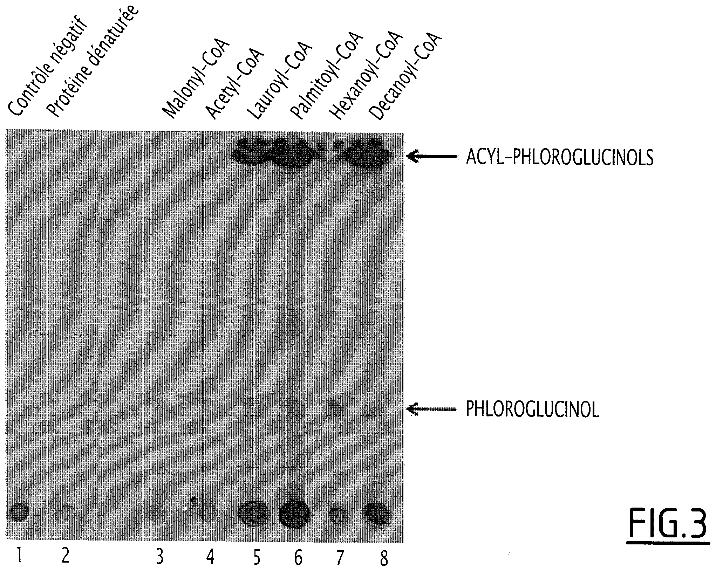

**Figure 3****.** La figure 3 représente l'analyse en chromatographie sur couche mince des produits formés au cours de la réaction enzymatique utilisant les différents substrats suivants : malonylCoA seul, malonylCoA + acétyl-CoA, malonylCoA + hexanoyl-CoA, malonylCoA + lauroyl-CoA, malonylCoA + palmytoyl-CoA et malonylCoA + décanoyl-CoA.

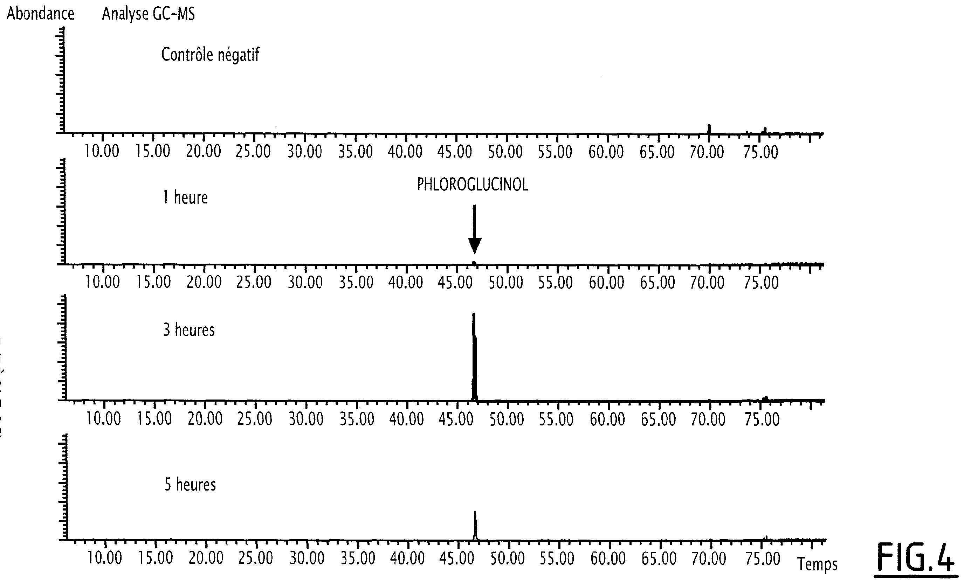

**Figure 4****.** La figure 4 représente l'analyse en spectrométrie de masse GC-MS des produits formés au cours de la réaction enzymatique utilisant les différents substrats suivants : malonylCoA seul, malonylCoA + acétyl-CoA, malonylCoA + hexanoyl-CoA, malonylCoA + lauroyl-CoA, malonylCoA + palmytoyl-CoA et malonylCoA + décanoyl-CoA.

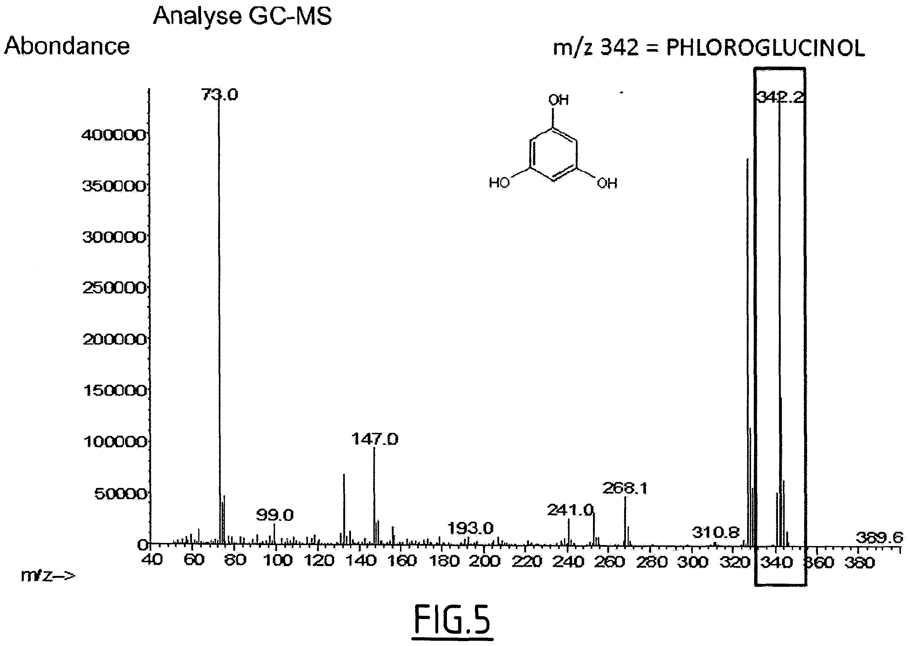

**Figure 5****.** La figure 5 représente l'analyse en spectrométrie de masse GC-MS des produits formés au cours de la réaction enzymatique utilisant les différents substrats suivants : malonylCoA seul, malonylCoA + acétyl-CoA, malonylCoA + hexanoyl-CoA, malonylCoA + lauroyl-CoA, malonylCoA + palmytoyl-CoA et malonylCoA + décanoyl-CoA.

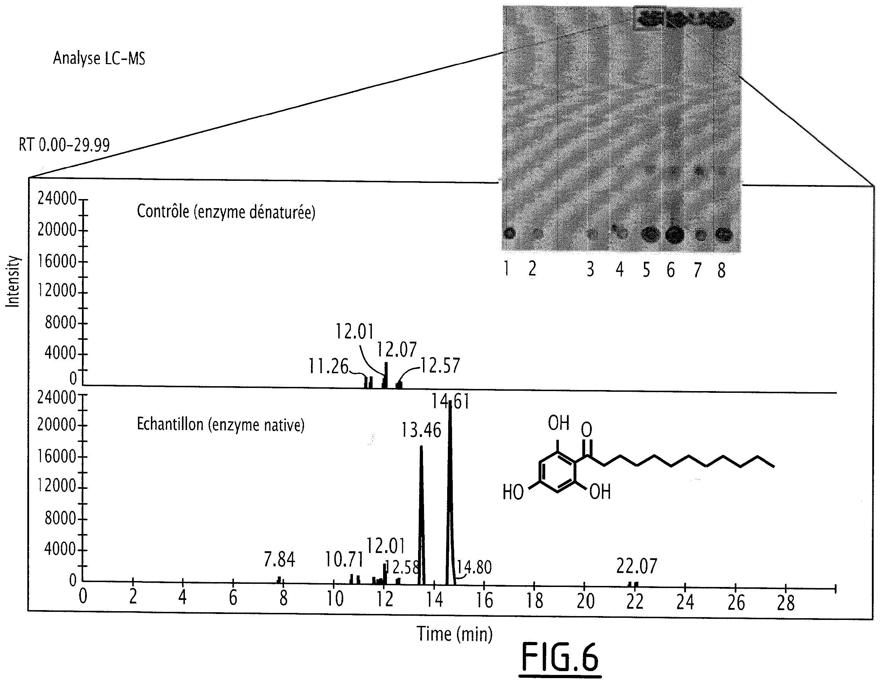

**Figure 6****.** La figure 6 représente l'analyse en spectrométrie de masse LC-MS des produits formés au cours de la réaction enzymatique en utilisant le malonylCoA seul ou le malonylCoA + acétyl-CoA. 1 : Contrôle négatif, 2 : Protéine dénaturée, 3 : Malonyl-CoA, 4 : Acétyl-CoA, 5 : Lauroyl-CoA, 6 : Palmitoyl-CoA, 7 : Hexanoyl-CoA, 8 : Decanoyl-CoA. Paramètres NL : 2.10^{E}4, m/z : 307.1877-307.1939, MS bl.

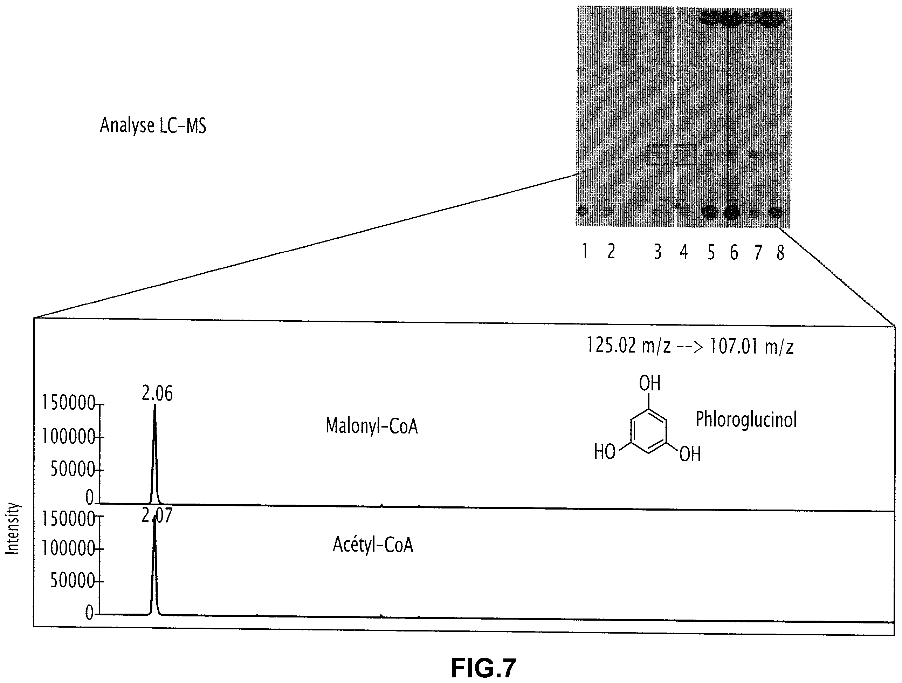

**Figure 7****.** La figure 7 représente l'analyse en spectrométrie de masse LC-MS des produits formés au cours de la réaction enzymatique en utilisant comme substrat le malonylCoA et le lauroyl-CoA. 1 : Contrôle négatif, 2 : Protéine dénaturée, 3 : Malonyl-CoA, 4 : Acétyl-CoA, 5 : Lauroyl-CoA, 6 : Palmitoyl-CoA, 7 : Hexanoyl-CoA, 8 : Decanoyl-CoA.

### EXEMPLES

### EXEMPLE 1 : Matériel, méthodes et procédures expérimentales

### 1.1. Produits chimiques

Les composés malonyl-CoA, acétyl-CoA, hexanoyl-CoA, lauroyl-CoA, palmytoyl-CoA et decanoyl-CoA proviennent de chez Sigma. Le [²⁻¹⁴C] Malonyl-CoA (55 mCi/mmol) provient de chez Perkin Elmer (USA).

### 1.2. Souche bactérienne

La souche *Escherichia coli* DH5a [*fhuA2 _(argF-lacZ)U169 phoA glnV44 Φ80 _(lacZ)M15 gyrA96 recA1 relA1 endA1 thi-1 hsdR17*] (Stratagene) a été utilisée en tant que souche hôte pour le maintien des plasmides. Pour l'expression protéique, les dérivés pQE-80L « cis-repressed » ont été transformés dans la souche *E. coli* BL21 (DE3) codon Plus RILP [*E. coli* B F- *ompT hsdS*(*rB*- mB-) *dcm*+ Tetr *gal* I (DE3) *endA* Hte *[argU ileY leuW* Camr]] (Stratagene) contenant des copies supplémentaires des ARNt arginine, isoleucine, proline et leucine.

### 1.3. Expression et purification de la polykétide synthase de type III recombinante d'Ectocarpus siliculosus

Le gène codant pour la PKS1 d'*E. siliculosus* a été amplifiée par PCR (30 cycles d'amplification) à partir de l'ADNc décrit dans Cock et al., 2010, Nature 465(7298): 617-621. Le vecteur d'expression pQE-80L (Qiagen) contenant le promoteur du bactériophage T5 inductible par l'Isopropyl-b-D-thiogalactopyranoside (IPTG) a été utilisé pour l'expression de la protéine (Figure 1). La PKS1 *E. siliculosus* a été clonée dans le vecteur au niveau des sites de restriction *Sph*I et *HindIII* en utilisant les oligonucléotides PQECHSFowBis (5'-GGCGGATCCGCATGCATGTCCAAGGACGAGCAGACGGTATACCCGGTCATCGCC-3'(SEQ ID NO : 11)) et PQECHSRev (5'-GGCTAAGCTTTTACTAGATCTGCCTGAGAAGGATGCCCTCTGCCCC-3' (SEQ ID NO : 12)). Les conditions de PCR utilisées pour le clonage sont les suivantes : 50 ng ADNc PKS1, 0,4 µM de chaque oligonucléotide, 0,4 mM dNTP mix dans un milieu réactionnel de 50 µL avec l'enzyme Phusion (Finnzyme) avec son tampon HF selon les recommandations du fournisseur. La réaction a été effectuée en 3 étapes : dénaturation de l'ADN et des amorces à 98°C pendant 5 min, 30 cycles de PCR à 98°C pendant 30s, 52°C pendant 30s, 72°C pendant 2min puis au final une étape de fin d'élongation à 72°C pendant 7min. Une étape supplémentaire consistant à ajouter des dA aux extrémités du fragment du PCR a été réalisée pendant 10min à 72°C avec l'ajout de 0,5 µL d'enzyme GoTaq (Promega). Le fragment d'ADN amplifié est purifié directement sur colonne MinElute (Qiagen). La ligation est effectuée dans un premier temps dans le pGEM-Teasy (Promega) selon des conditions standards (une nuit à température ambiante dans un milieu réactionnel de 12 µL contenant un ratio 10/1 de produit à cloner/vecteur avec la T4 DNA ligase du kit Promega). Le produit de ligation est introduit par choc thermique dans des cellules compétentes DH5alpha préparées au laboratoire, et un transformant est sélectionné pour la production en masse de vecteur recombinant contenant l'insert PKS1. Le plasmide est purifié avec le kit Miniprep SV (Promega). Après vérification de la séquence nucléotidique de PKS1, l'insert est digéré par Sphl et Hindlll, puis purifié sur gel d'agarose avec le kit MinElute (Qiagen) avant d'être ligué dans le vecteur pQE80L (Qiagen) doublement digéré avec les mêmes enzymes de restriction et déphosphorylé par la SAP (NE Biolabs). Les conditions de ligation dans pQE80L sont les mêmes que décrites précédemment.

Les constructions codent pour une protéine totale à laquelle une étiquette de six histidines (His-tag) a été ajoutée à son extrémité N-terminale. Les constructions ont été transformées dans la souche *E. coli* BL21-CodonPlus-RILP strain (Stratagene) en utilisant un milieu LB solide contenant 100 µg/mL d'ampicilline. L'expression de la protéine recombinante a été réalisée en cultivant les bactéries dans un milieu ZYP à 20°C en utilisant un lot fermenteur de 5L. Après 48 heures de culture, l'induction de l'expression protéique est poursuivie en ajoutant 0,5 mM d'IPTG. Après cette dernière induction, les cellules sont collectées par centrifugation et congelées à -80°C.

Les cellules sont ensuite remises en suspension dans le milieu A (20 mM Tris-HCl pH 7,5, 300 mM NaCl et 50 mM Imidazole) supplémenté avec un mélange d'inhibiteur de protéases, du lysozyme (1mg/mL) et de la DNase (10 mg/ml). La lyse des cellules est ensuite réalisées par deux passages dans une presse de French afin de réduire la viscosité du surnageant. Les débris cellulaires sont éliminés par centrifugation à 20000 t/min à 4°C pendant 1h30. Le surnageant est ensuite transféré dans une colonne Ni-sepharose (GE Healthcare). L'extrait cellulaire est ensuite fractionné par chromatographie d'affinité dite « IMAC » (Immobilized-metal affinity chromatography) sur un appareil ÄKTA ™-Avant (GE Healthcare). Après une étape de lavage avec un tampon A (20 mM Tris-HCl pH 7.5; 300 mM NaCl ; 50 mM imidazole), les protéines sont éluées selon un protocole de gradient de 50 mM à 500 mM d'imidazole en mélangeant le tampon A avec le tampon B (20 mM Tris-HCl pH 7,5; 300 mM NaCl ; 500 mM imidazole). Les fractions C12 à E10 ont ensuite été concentrées à 5 ml par ultrafiltration sur un CentriPrep 10 kDa (Millipore) et simultanément échangées dans un tampon contenant 20 mM Tris-HCl pH 7,5 100 mM NaCl.

Les protéines sont ensuite transférées dans une colonne de filtration sur gel Superdex S-200 HR 16/60 (GE Healthcare) et purifiées par chromatographie d'exclusion stérique en utilisant un appareil ÄKTA-Avant (GE Healthcare). La pureté et l'intégrité de tous les échantillons de protéines ont été analysées par électrophorèse sur gel 12%SDS-polyacrylamide et par spectrométrie de masse MALDI-TOF.

### 1.4. Essais enzymatiques (Chromatographie sur couche mince (CCM))

Les essais enzymatiques ont été réalisés en utilisant :
- a) le malonyl-CoA seul : 200µM de malonyl-CoA a été ajouté au mélange d'essai contenant 20 µM de Malonyl-CoA radio-marqué au [²⁻¹⁴C] (55 mCi/mmol), 50 µg d'enzyme recombinante purifiée PKS1 d'*E*. *siliculosus,* dans un volume final de 500 µl de 50 mM Tris HCI pH 7,5 et 1 mM d'EDTA, ou
- b) les cinq substrats suivants : acétyl-CoA, hexanoyl-CoA, lauroyl-CoA, palmytoyl-CoA et décanoyl-CoA. 200 µM de chaque substrat a été ajouté au mélange d'essai contenant 20 µM de Malonyl-CoA radio-marqué au [2-14C] (55 mCi/mmol), 50 µg d'enzyme recombinante purifiée PKS1 d'*E*. *siliculosus,* dans un volume final de 500 µl de 50 mM Tris HCI pH 7,5 et 1 mM d'EDTA.

L'incubation des mélanges en a) et b) a été réalisée à température ambiante pendant 1h ou 3h et a été stoppée par l'ajout de HCl à 37%. Les produits des réactions ont été ensuite extraits avec 1 ml d'acétate d'éthyl, et séparés par chromatographie sur couche mince (Merck Art. 1.11798 Silica gel 60 F254; ethyl acetate/hexane/AcOH 65 : 25 : 5, v/v/v).

Les signaux radioactifs ont été détectés et quantifiés à l'aide d'un système d'imagerie Typhoon (Molecular Dynamics-GE Healthcare).

### 1.5. Essais enzymatiques (Spectrométrie de masse couplée à la chromatographie en phase gazeuse GC-MS)

Les essais enzymatiques ont été réalisés en utilisant :
- a) le malonyl-CoA seul : 200µM de malonyl-CoA a été ajouté au mélange d'essai contenant 50 µg d'enzyme recombinante purifiée PKS1 d'*E*. *siliculosus,* dans un volume final de 500 µl de 50 mM Tris HCI pH 7,5 et 1 mM d'EDTA, ou
- b) les cinq substrats suivants : acétyl-CoA, hexanoyl-CoA, lauroyl-CoA, palmytoyl-CoA et décanoyl-CoA. 200 µM de chaque substrat a été ajouté au mélange d'essai contenant 20 µM de Malonyl-CoA, 50 µg d'enzyme recombinante purifiée PKS1 d'*E*. *siliculosus,* dans un volume final de 500 µl de 50 mM Tris HCI pH 7,5 et 1mM d'EDTA.

L'incubation des mélanges en a) et b) a été réalisée à température ambiante pendant 1, 2, 3, 4, ou 5h et a été stoppée par l'ajout de HCI à 37%. Les produits des réactions ont été ensuite extraits avec 1 ml d'acétate d'éthyle. 2,50 µg de vanilline ont été ajouté en tant que standard interne. Les échantillons ont ensuite été vortexés pendant 5 min et centrifugés pendant 5 min à 1000g. La phase organique a été transférée dans un flacon en verre et évaporée sous un flux d'azote. Des triméthylsilyl-éthers (TMS-ether) ont été formés par addition de 100 µl d'acétonitrile et de 100 µl de Sylon-BFT pendant 60 min à 60°C et évaporés sous un flux d'azote. Les métabolites sont resuspendus dans 100 µl d'hexane et analysés par GC-MS en mode El sur un Agilent GC 6890 couplé à un détecteur « 5973 MS Detector » (Agilent, Les Ulis, France) et équipé d'une colonne DB-5MS (30 m × 0.25 mm de diamètre interne × 0,25 µm d'épaisseur de film (J and W Scientific, Agilent)). Les températures de l'orifice d'injection et de l'interface sont respectivement 250 et 280°C; celle de la source d'ion et de l'analyseur MS ont été fixées respectivement à 230 et 150°C. Les échantillons ont été injectés en mode « d'injection sans division » ou mode « splitless ». La température du four a été premièrement fixée à 60°C pendant 5 min, puis augmentées de 10°C/min jusqu'à 100°C, élevée de 1°C/min jusqu'à 150°C et finalement élevée de 8°C/min jusqu'à 290°C, puis maintenue pendant 5 min. Les composés ont été ionisés par impact électronique à 70eV d'énergie. Les analytes ont été détectés par un courant ionique total de 50 à 850 m/z. Toutes les données ont été traitées avec le logiciel MSDchem (EMBL-EBI).

### 1.6. Essais enzymatiques (spectrométrie de masse couplée à la chromatographie en phase liquide LC-MS)

Les essais enzymatiques ont été réalisés en utilisant :
- a) le malonyl-CoA seul : 200µM de malonyl-CoA a été ajouté au mélange d'essai contenant 50 µg d'enzyme recombinante purifiée PKS1 d'*E*. *siliculosus,* dans un volume final de 500 µl de 50 mM Tris HCI pH 7,5 et 1 mM d'EDTA, ou
- b) les cinq substrats suivants : acétyl-CoA, hexanoyl-CoA, lauroyl-CoA, palmytoyl-CoA et décanoyl-CoA. 200 µM de chaque substrat a été ajouté au mélange d'essai contenant 20 µM de Malonyl-CoA, 50 µg d'enzyme recombinante purifiée PKS1 d'*E*. *siliculosus,* dans un volume final de 500 µl de 50 mM Tris HCI pH 7,5 et 1mM d'EDTA.

L'incubation des mélanges en a) et b) a été réalisée à température ambiante pendant 1, 2, 3, 4, ou 5h et a été stoppée par l'ajout de HCI à 37%. Les produits des réactions ont été ensuite extraits avec 1 ml d'acétate d'éthyle. 2,50 µg de vanilline ont été ajouté en tant que standard interne. Les échantillons ont ensuite été vortexés pendant 5 min et centrifugés pendant 5 min à 1000g. La phase organique a été transférée dans un flacon en verre et évaporée sous un flux d'azote. Les métabolites sont resuspendus dans 100 µl d'hexane. Pour l'analyse LC-MS, un système de chromatographie liquide DionexUltiMate 3000Rapid Separation LC (RSLC, Dionex) est couplé à un spectromètre de masse hybride LTQ-Orbitrap™ (Thermo Fisher Scientific). Tous les solvants et réactifs utilisés sont de qualité analytique ou HPLC grade (Carlo Erba). La séparation chromatographique a été réalisée sur une colonne Acclaim RSLC 120, C18, 2 µm taille de particules, 2,1 x 100 mmcolumn (Dionex) maintenue à 20°C. La phase mobile est de l'eau contenant 0,1% d'acide acétique (A) et de l'acétonitrile contenant 0,1% d'acide acétique (B). Le flux a été réglé à 0,25 ml.min-1. Le gradient d'élution (A:B, v/v) a été réalisé comme décrit en suivant: 80:20 de 0 à 1 min; puis 0:100 de 1 à 10 min; puis 20:100 pendant 10 min et à 20,1 min à 80:20 pour 10 min. Le volume injecté est de 50 µL. La colonne HPLC a été connectée sans découplage avec l'interface opérative électrospray en mode négatif. Le voltage du spray était de 3,5 kV et la température du capillaire de transfert a été maintenue à 350 °C. Le "sheath liquid" et l'azote gazeux auxiliaire ont été appliqués pour aider à l'évaporation de solvant à un flux de 25 et 5 unités arbitraires respectivement. La totalité des scans des spectres de masse a été obtenue pour des m/z de 50 à 2000 en utilisant une résolution de masse de 30,000 FWHM à 400 m/z en mode profil.

### EXEMPLE 2 : Résultats

### 2.1. Séquences nucléotidique et protéique de PKS1

Le gène *PKS1* présent sur le génome d'*E*. *siliculosus* est disponible dans les bases de données publiques depuis juin 2010 et il correspond à une séquence de 1245 nucléotides, codant pour une protéine de 415 acides aminés. Un peptide signal d'adressage a été prédit selon l'utilisation du logiciel SIGNALP v.2.0 qui utilise les deux modèles Neural Networks et Hidden Markov (Nielsen et al., Protein Eng, 1999, 12 : 3-9) et cette séquence de 102 nucléotides, correspondant aux 34 premiers acides aminés, a été éliminée pour donner une protéine recombinante mature exprimée dans le cytoplasme bactérien.

### 2.2. Surexpression chez E.coli et purification de la PKS1 recombinante

Suivant les protocoles de surexpression et de purification de PKS1 chez *E. coli,* le gel d'électrophorèse des fractions d'élution de la colonne d'affinité « IMAC » coloré au bleu de Coomassie a permis de détecter la production d'une protéine recombinante à la taille attendue d'environ 50 kDa. Pour augmenter le niveau de pureté de la protéine recombinante et éliminer les formes inactives de l'enzyme (*e.g*. agrégats solubles), une seconde purification sur une colonne d'exclusion de taille a permis d'obtenir la protéine recombinante avec une homogénéité à plus de 99% selon une analyse en DLS (Dynamic Light Scattering). Cette analyse coïncide avec le volume d'élution de la protéine sur gel filtration et suggère que la forme active de l'enzyme est bien un dimère comme la majorité des PKS III connues à ce jour. La pureté de l'enzyme a aussi été validée par une analyse en spectrométrie de masse MALDI-TOF de la bande découpée sur gel et digérée à la trypsine. Les masses des fragments obtenus correspondent bien à la séquence de PKS1 avec 32 peptides identifiés correspondant à une couverture de 47% de la séquence (Figure 2).

Le rendement de production pour une culture en fermenteur de 5L a été estimé à environ 5 à 10 mg de protéine recombinante active par litre de culture et peut donc supporter un niveau de production industriel. De plus, l'enzyme s'est révélée stable pendant un à deux mois à 4°C et elle supporte la congélation à -80°C.

### 2.3. Activité enzymatique : analyses en spectrométrie de masse des composés formés

L'analyse des produits formés en CCM indique qu'il y a formation de phloroglucinol avec ou sans starters (malonylCoA seul, malonylCoA + acétyl-CoA, malonylCoA + hexanoyl-CoA, malonylCoA + lauroyl-CoA, malonylCoA + palmytoyl-CoA et malonylCoA + décanoyl-CoA) et que les composés principaux de la réaction obtenus sont le phloroglucinol dans le cas du malonylCoA seul ou du malonylCoA + acétyl-CoA et un acyl-phloroglucinol dans le cas du malonylCoA avec les autres starters (acétyl-CoA, hexanoyl-CoA, lauroyl-CoA, palmytoyl-CoA et décanoyl-CoA) (Figure 3).

Ces résultats ont été confirmés par l'analyse en spectrométrie de masse GC-MS qui a permis d'identifier clairement la production de phloroglucinol à partir de malonyl-CoA avec un optimum de production situé entre 3h et 5h de réaction (Figures 4 et 5 et Tableau 5).

**Tableau 5 : Quantité de phloroglucinol produite par la PKS1 à partir de Malonyl-CoA.**

| Temps (h) | Aire m/z 194 | Aire *m*/*z* 342 | Rapport 342/194 | Phloroglucinol/ échantillon (µg) |
|---|---|---|---|---|
| **CB** | 6232490 | 0 | 0 | **0,00** |
| **1** | 5968432 | 955510 | 0,16009397 | **1,70** |
| **2** | 6169748 | 5641093 | 0,91431498 | **9,08** |
| **3** | 5847716 | 39184281 | 6,70078386 | **65,64** |
| **4** | 4159877 | 9789969 | 2,35342752 | **23,15** |
| **5** | 4452610 | 12232641 | 2,74729675 | **27,00** |

L'analyse en spectrométrie de masse LC-MS a également permis d'identifier clairement la production de phloroglucinol à partir de malonyl-CoA et d'acétyl-CoA (Figure 6).

La formation des acyl-phloroglucinols a été mise en évidence en extrayant spécifiquement les composés présents sur CCM et en les analysant par spectrométrie de masse LC-MS. Ainsi, par exemple, en présence de malonylCoA et lauroyl-CoA l'enzyme PKS1 produit le lauroyl-phloroglucinol (Figure 7).

D'autres résultats d'analyse en GC-MS de produits formés en présence des différents acyl-CoA suggèrent aussi la formation de composés de type tétraketide pyrones comme chez *Mycobacterium tuberculosis.*

### 2.4. Cristallisation et obtention de la structure de l'enzyme PKS1

Après obtention des conditions de cristallisation, plusieurs cristaux de l'enzyme PKS1 ont permis d'obtenir des jeux de données de diffraction aux rayons X permettant la résolution de la structure à 2,85 Å par la technique de remplacement moléculaire (Tableau 6).

**Tableau 6. Données collectées et statistiques de pureté de la PKS à la résolution de 2,85 Å.**

| **Collecte des données** | **PKS Haute résolution** |
|---|---|
| Ligne de faisceau ESRF | ID23-1 |
| Longueur d'onde (Å) | 0.979239 |
| Groupe d'espace | P2₁2₁2₁ |
| Paramètres de la maille (Å) | A=61.99,b=83.92, c=154.91 |
| Résolution (Å) | 83,920-2,85 (3.02-2.85) |
| Nombre d'observations (F>0) | 93096 (12976) |
| Nombre de réflections uniques | 22949 (3105) |
| Achèvement (%) | 100 (100) |
| Moyenne I/ (I) | 3,8 (1,4) |
| Rₚᵢₘ (%) | 13,3 (58,7) |
| Redondance | 4,1 (4,2) |

| **Affinement** | |
|---|---|
| Résolution | 56,917-2,85 |
| Nombre de réflections uniques | 21715 |
| Facteur R (R_{free} on 5%) | 30,17 (41,31) |
| Nombre d'atomes/protéine (moyenne facteur B en Å²) | 5768 (45,86) |
| Nombre d'atomes de solvant (moyenne facteur B en Å²) | 4 (34,20) |
| Ecart-type sur les liaisons (Å) | 0,013 |
| Ecart-type sur les angles (°) | 1,736 |
| Moyenne Bfact (Å²) | 45,769 |

Ces données confirment la formation d'un dimère de PKS1 pour l'enzyme sous sa forme recombinante active.

La structure globale d'un monomère de PKS1 révèle une composition en hélices α et feuillets β organisés en un repliement canonique de type αβαβα-thiolase. La triade catalytitique est représentée par les résidus Cys194, His331 et Asn364. Comme la PKS III de *Mycobacterium tuberculosis* nommée PKS18, PKS1 semble présenter un tunnel d'accès au site catalytique où peuvent s'engouffrer des acyl-CoA à plus ou moins longues chaînes. Le site de fixation potentiel du malonyl-CoA a aussi été identifié.

## Revendications

1. Méthode de production d'au moins un composé polyphénolique, dans laquelle :
- une polyketide synthase de type III (PKSIII) d'algue brune est mise en contact avec au moins un substrat carboné dans des conditions permettant la production majoritaire d'au moins un composé polyphénolique, **caractérisée en ce que** ladite PKSIII comprend ou consiste en :
- la séquence d'acides aminés SEQ ID NO : 1 ou une séquence ayant au moins 93% d'identité avec SEQ ID NO : 1, ou une séquence susceptible d'être codée par la séquence nucléotidique SEQ ID NO : 2 ou une séquence ayant au moins 85% d'identité avec SEQ ID NO : 2, ou
- la séquence d'acides aminés SEQ ID NO : 3 ou une séquence ayant au moins 93% d'identité avec SEQ ID NO : 3, ou une séquence susceptible d'être codée par la séquence nucléotidique SEQ ID NO : 4 ou une séquence ayant au moins 85% d'identité avec SEQ ID NO : 4.
- ledit composé phénolique produit est le phloroglucinol ou un de ses dérivés,
- ledit substrat est sélectionné parmi le malonyl-CoA, l'hexanoyl-CoA, le decanoyl-CoA, le lauroyl-CoA et/ou le palmitoyl-CoA.

2. Méthode selon la revendication 1, **caractérisée en ce que** ledit substrat est le malonyl-CoA et ledit composé phénolique produit est le phloroglucinol.

3. Méthode selon l'une quelconque des revendications 1 ou 2, **caractérisée en ce que** ledit substrat est le malonyl-CoA et le lauroyl-CoA et ledit composé phénolique produit est le lauroyl-phloroglucinol.

4. Méthode selon l'une quelconque des revendications 1 à 3, dans laquelle ladite PKSIII est obtenue après les étapes de :
- culture d'une cellule hôte recombinante comprenant un vecteur recombinant codant pour une PKSIII comprenant ou consistant en (i) la séquence d'acides aminés SEQ ID NO : 1 ou une séquence ayant au moins 93% d'identité avec SEQ ID NO : 1, ou un vecteur recombinant comprenant la séquence nucléotidique SEQ ID NO : 2 ou une séquence ayant au moins 85% d'identité avec SEQ ID NO : 2, ou (ii) la séquence d'acides aminés SEQ ID NO : 3 ou une séquence ayant au moins 93% d'identité avec SEQ ID NO : 3, ou un vecteur recombinant comprenant la séquence nucléotidique SEQ ID NO : 4 ou une séquence ayant au moins 85% d'identité avec SEQ ID NO : 4 dans des conditions permettant l'expression de ladite PKSIII, et
- extraction et/ou purification, de ladite PKSIII.

5. Acide nucléique isolé consistant en la séquence SEQ ID NO : 2 ou SEQ ID NO : 4.

6. Acide nucléique isolé consistant en une séquence au moins 85% identique à la séquence SEQ ID NO : 2 ou au moins 95 % identique à la séquence SEQ ID NO : 4, **caractérisé en ce qu'**il code pour une polyketide synthase de type III (PKSIII).

7. Acide nucléique isolé selon la revendication 5, codant pour une polyketide synthase de type III (PKSIII).

8. Acide nucléique isolé comprenant ou consistant en la séquence complémentaire de SEQ ID NO : 2 ou SEQ ID NO : 4.

9. Acide nucléique isolé codant pour une polyketide synthase de type III (PKSIII) consistant en :
a) la séquence nucléotidique SEQ ID NO : 2,
b) la séquence nucléotidique SEQ ID NO : 4,
c) la séquence complémentaire de SEQ ID NO : 2 ou SEQ ID NO : 4,
d) une séquence au moins 85% identique à SEQ ID NO : 2 ou au moins 95 % identique à la séquence SEQ ID NO : 4,
e) une séquence différant des séquences a) à d) par dégénérescence du code,
f) une séquence nucléotidique s'hybridant en conditions de stringence spécifiques avec au moins l'une des séquences a) à e).

10. Polyketide synthase isolée **caractérisée en ce qu'**elle est codée par un acide nucléique isolé selon l'une quelconque des revendications 5 à 9.

11. Polyketide synthase isolée **caractérisée en ce que** ladite protéine consiste en :
- la séquence SEQ ID NO : 1 ou une séquence ayant au moins 93% d'identité avec la SEQ ID NO : 1, ou
- la séquence SEQ ID NO : 3 ou une séquence ayant au moins 93% d'identité avec la SEQ ID NO : 3.

12. Vecteur recombinant, comprenant l'acide nucléique isolé selon l'une quelconque des revendications 5 à 9.

13. Cellule hôte recombinante, **caractérisée en ce que** la cellule a été transformée par un vecteur recombinant selon la revendication 12.

14. Méthode de production d'une polyketide synthase, comprenant les étapes de :
a) culture d'une cellule selon la revendication 13, dans des conditions permettant l'expression d'une polyketide synthase recombinante,
b) extraction et/ou purification, de ladite polyketide synthase recombinante.

## Patentansprüche

1. Verfahren zur Herstellung von mindestens einer Polyphenolzusammensetzung, wobei:
- einer Typ-III-Polyketid-Synthase (PKSIII) aus Meeresbraunalge mit mindestens einem kohlenstoffhaltigen Substrat unter Bedingungen in Kontakt gebracht wird, die die mehrheitliche Herstellung von mindestens einer Polyphenolzusammensetzung ermöglichen, **dadurch gekennzeichnet, dass** die PKSIII umfasst oder besteht aus:
der Aminosäuresequenz SEQ ID Nr.: 1 oder einer Sequenz, aufweisend mindestens 93 % Identität mit SEQ ID Nr.: 1, oder eine Sequenz, die durch die Nukleotidsequenz SEQ ID Nr.: 2 codiert werden kann, oder einer Sequenz, aufweisend mindestens 85 % Identität mit SEQ ID Nr.: 2, oder
- der Aminosäuresequenz SEQ ID Nr.: 3 oder einer Sequenz, aufweisend mindestens 93 % Identität mit SEQ ID Nr.: 3, oder eine Sequenz, die durch die Nukleotidsequenz SEQ ID Nr.: 4 codiert werden kann, oder eine Sequenz, aufweisend mindestens 85 % Identität mit SEQ ID Nr.: 4,
- wobei die erzeugte Phenolzusammensetzung Phloroglucinol oder eines seiner Derivate ist,
- wobei das Substrat ausgewählt ist aus Malonyl-CoA, Hexanoyl-CoA, Decanoyl-CoA, Lauroyl-CoA und/oder Palmitoyl-CoA.

2. Verfahren nach Anspruch 1, **dadurch gekennzeichnet, dass** das Substrat Malonyl-CoA ist und die erzeugte Phenolzusammensetzung Phloroglucinol ist.

3. Verfahren nach einem der Ansprüche 1 oder 2, **dadurch gekennzeichnet, dass** das Substrat Malonyl-CoA und Lauroyl-CoA ist und die erzeugte Phenolzusammensetzung Lauroyl-Phloroglucinol ist.

4. Verfahren nach einem beliebigen der Ansprüche 1 bis 3, wobei die PKSIII nach den folgenden Schritten erhalten wird:
- Kultivieren einer rekombinanten Wirtszelle, umfassend einen rekombinanten Vektor, der für eine PKSIII codiert, umfassend oder bestehend aus (i) der Aminosäuresequenz SEQ ID Nr.: 1 oder eine Sequenz, aufweisend mindestens 93 % Identität mit SEQ ID Nr.: 1, oder einen rekombinanten Vektor, umfassend die Nukleotidsequenz SEQ ID Nr.: 2 oder eine Sequenz, aufweisend mindestens 85 % Identität mit SEQ ID Nr.: 2, oder (ii) der Aminosäuresequenz SEQ ID Nr.: 3 oder eine Sequenz, aufweisend mindestens 93 % Identität mit SEQ ID Nr.: 3, oder einen rekombinanten Vektor, umfassend die Nukleotidsequenz SEQ ID Nr.: 4 oder eine Sequenz, aufweisend mindestens 85 % Identität mit SEQ ID Nr.: 4 unter Bedingungen, die die Expression der PKSIII ermöglichen, und
- Extrahieren und/oder Reinigen der PKSIII.

5. Isolierte Nukleinsäure, bestehend aus der Sequenz SEQ ID Nr.: 2 oder SEQ ID Nr.: 4.

6. Isolierte Nukleinsäure, bestehend aus einer Sequenz mit mindestens 85 % Identität mit der Sequenz SEQ ID Nr.: 2 oder mindestens 95 % Identität mit der Sequenz SEQ ID Nr.: 4, **dadurch gekennzeichnet, dass** sie für eine Typ-III-Polyketid-Synthase (PKSIII) codiert.

7. Isolierte Nukleinsäure nach Anspruch 5, die für eine Typ-III-Polyketid-Synthase (PKSIII) codiert.

8. Isolierte Nukleinsäure, umfassend oder bestehend der komplementären Sequenz von SEQ ID Nr.: 2 oder SEQ ID Nr.: 4.

9. Isolierte Nukleinsäure, die für eine Typ-III-Polyketid-Synthase (PKSIII) codiert, bestehend aus:
a) der Nukleotidsequenz SEQ ID Nr.: 2,
b) der Nukleotidsequenz SEQ ID Nr.: 4,
c) der komplementären Sequenz von SEQ ID Nr.: 2 oder SEQ ID Nr.: 4,
d) einer Sequenz mit mindestens 85 % Identität mit der Sequenz SEQ ID Nr.: 2 oder mindestens 95 % Identität mit der Sequenz SEQ ID Nr.: 4,
e) einer Sequenz, die verschieden von den Sequenzen a) bis d) durch Degradation des Codes ist,
f) einer Nukleodidsequenz, die sich unter spezifischen Stringenzbedingungen mit mindestens einer der Sequenzen a) bis e) hybridisiert.

10. Isolierte Polyketid-Synthase, **dadurch gekennzeichnet, dass** sie durch eine isolierte Nukleinsäure nach einem beliebigen der Ansprüche 5 bis 9 codiert ist.

11. Isolierte Polyketid-Synthase, **dadurch gekennzeichnet, dass** das Protein aus Folgendem besteht:
- der Sequenz SEQ ID Nr.: 1 oder einer Sequenz, aufweisend mindestens 93 % Identität mit der SEQ ID Nr.: 1, oder
- der Sequenz SEQ ID Nr.: 3 oder einer Sequenz, aufweisend mindestens 93 % Identität mit SEQ ID Nr.: 3.

12. Rekombinanter Vektor, umfassend die isolierte Nukleinsäure Verfahren nach einem beliebigen der Ansprüche 5 bis 9.

13. Rekombinante Wirtszelle, **dadurch gekennzeichnet, dass** die Zelle durch einen rekombinanten Vektor nach Anspruch 12 umgewandelt wurde.

14. Verfahren zur Herstellung einer Polyketid-Synthase, umfassend die folgenden Schritte:
a) Kultivieren der Wirtszelle nach Anspruch 13 unter Bedingungen, die die Expression einer rekombinanten Polyketid-Synthase ermöglichen,
b) Extrahieren und/oder Reinigen der rekombinanten Polyketid-Synthase.

## Claims

1. A method for producing at least one polyphenol compound, wherein:
- a polyketide synthase of type III (PKSIII) from brown algae is put into contact with at least one carbonaceous substrate under conditions allowing majority production of at least one polyphenol compound, **characterized in that** said PKSIII comprises or consists in :
- the aminoacid sequence SEQ ID NO: 1 or a sequence having at least 93% identity with SEQ ID NO: 1, or a sequence which may be coded by the nucleotide sequence SEQ ID NO: 2 or a sequence having at least 85% identity with SEQ ID NO: 2, or
- the aminoacid sequence SEQ ID NO: 3 or a sequence having at least 93% identity with SEQ ID NO: 3, or a sequence which may be coded by the nucleotide sequence SEQ ID NO: 4 or a sequence having at least 85% identity with SEQ ID NO: 4
- said produced phenol compound is phloroglucinol or one of its derivatives?
- said substrate is selected from malonyl-CoA, hexanoyl-CoA, decanoyl-CoA, lauroyl-CoA and/or palmitoyl-CoA.

2. The method according to claim 1, **characterized in that** said substrate is malonyl-CoA and said produced phenol compound is phloroglucinol.

3. The method according to anyone of claim 1 or 2, **characterized in that** said substrate is malonyl-CoA and lauroyl-CoA and said produced phenol compound is lauroyl-phloroglucinol.

4. The method according to any of claims 1 to 3, wherein said PKSIII is obtained after the steps of:
- cultivating a recombinant host cell comprising a recombinant vector coding for a PKSIII comprising or consisting in (i) the aminoacid sequence SEQ ID NO: 1 or having at least 93% identity with SEQ ID NO: 1, or a recombinant vector comprising the nucleotide sequence SEQ ID NO: 2 or a sequence having at least 85% identity with SEQ ID NO: 2, or (ii) the aminoacid sequence SEQ ID NO: 3 or a sequence having at least 93% identity with SEQ ID NO: 3, or a recombinant vector comprising the nucleotide sequence SEQ ID NO: 4 or a sequence having at least 85% identity with SEQ ID NO: 4 under conditions allowing expression of said PKSIII, and
- extracting and/or purifying said PKSIII.

5. An isolated nucleic acid consisting in the sequence SEQ ID NO: 2 or SEQ ID NO: 4.

6. An isolated nucleic acid consisting in a sequence at least 85% identical with the sequence SEQ ID NO: 2 or at least 95% identical with SEQ ID NO: 4, characterize din that it codes for a for a polyketide synthase of type III (PKSIII).

7. The isolated nucleic acid according to claim 5 coding for a polyketide synthase of type III (PKSIII).

8. An isolated nucleic acid comprising or consisting in the complementary sequence of SEQ ID NO: 2 or SEQ ID NO: 4.

9. An isolated nucleic acid coding for a polyketide synthase of type III (PKSIII) consisting in:
a) the nucleotide sequence SEQ ID NO: 2,
b) the nucleotide sequence SEQ ID NO: 4,
c) the complementary sequence of SEQ ID NO: 2 or SEQ ID NO: 4,
d) a sequence at least 85% identical with SEQ ID NO: 2 or at least 95% identical with SEQ ID NO: 4,
e) a sequence differing from the sequences a) to d) by degeneration of the code,
f) a nucleotide sequence hybridizing on the specific stringency conditions with at least one of the sequences a) to e).

10. An isolated polyketide synthase **characterized in that** it is coded by an isolated nucleic acid according to any of claims 5 to 9.

11. An isolated polyketide synthase **characterized in that** said protein consists in:
- the sequence SEQ ID NO: 1 or a sequence having at least 93% identity with SEQ ID NO: 1, or
- the sequence SEQ ID NO: 3 or a sequence having at least a 93% identity with SEQ ID NO: 3.

12. A recombinant vector, comprising the isolated nucleic acid according to any of claims 5 to 9.

13. A recombinant host cell, **characterized in that** the cell has been transformed with a recombinant vector according to claim 12.

14. A method for producing a polyketide synthase, comprising the steps of:
a) cultivating a cell according to claim 13, under conditions allowing expression of a recombinant polyketide synthase,
b) extracting and/or purifying said recombinant polyketide synthase.


### SEQUENCE LISTING

<110> CENTRE NATIONAL DE LA RECHERCHE SCIENTIFIQUE (C.N.R.S) UNIVERSITE PIERRE ET MARIE CURIE (UPMC)
<120> UTILISATION DE POLYKETIDE SYNTHASES DE TYPE III (PKS III) RECOMBINANTES D'ALGUES BRUNES MARINES
<130> BET12P2509
<150> FR 1158728
   <151> 2011-09-29
<160> 12
<170> PatentIn version 3.5
<210> 1
   <211> 290
   <212> PRT
   <213> Ectocarpus siliculosus
<400> 1
   Val Leu Glu Arg Ile Tyr Gly Asn Ser Arg Ile Gly Ser Arg Tyr Phe
   1               5                   10                  15
   Ala Val Pro Asp Phe Thr Pro Gly Arg Ala Ala Lys Gly Asp Pro Leu
               20                  25                  30
   Phe Tyr Pro Ala Asp Gly Ser Tyr Gln Val Pro Val Asp Val Arg Leu
           35                  40                  45
   Asp Lys Phe Lys Glu Lys Ala Val Pro Leu Val Ser Asp Val Ala Arg
       50                  55                  60
   Arg Ala Ile Lys Glu Ala Gly Leu Asn Val Glu Asp Ile Ser Lys Leu
   65                  70                  75                  80
   Val Val Val Ser Ser Thr Gly Phe Leu Gly Pro Gly Leu Asp Cys Glu
                   85                  90                  95
   Leu Ile Lys Asn Leu Gly Leu Thr Arg Ser Val Asp Arg Thr Leu Ile
               100                 105                 110
   Gly Phe Met Gly Cys Ala Ala Ala Met Asn Gly Phe Arg Asn Ala Asn
           115                 120                 125
   Asp Tyr Val Thr Ala Asn Pro Gly Lys Tyr Ala Leu Met Ile Cys Val
       130                 135                 140
   Glu Leu Ser Ser Val His Thr Thr Phe Asp Asp Asn Ile Asn Asp Ala
   145                 150                 155                 160
   Ile Leu His Ala Ile Phe Ala Asp Gly Cys Ala Ala Ala Val Leu Lys
                   165                 170                 175
   Gly Ala Arg Lys Ser Glu Cys Pro Lys Gly Thr Leu Ala Ile Val Asp
               180                 185                 190
   Asn His Ala Trp Leu Met Glu Gly Thr Glu Asp Gly Ile Thr Leu Ala
           195                 200                 205
   Ile Lys Pro Asn Gly Ile Thr Cys Thr Leu Ser Lys Phe Leu Pro Gln
       210                 215                 220
   Tyr Ile Ala Lys Asn Ile Ala Phe Phe Ala Asp Gly Phe Leu Lys Lys
   225                 230                 235                 240
   His Lys Leu Gly Arg Asp Asp Val Asp Phe Trp Cys Val His Pro Gly
                   245                 250                 255
   Gly Arg Arg Ile Ile Glu Glu Ala Gln Asn Gly Leu Gly Leu Ser Glu
               260                 265                 270
   Glu Gln Thr Ala Asp Ser Trp Ala Val Leu Gly Glu Tyr Gly Asn Met
           275                 280                 285
   Leu Ser
       290
<210> 2
   <211> 870
   <212> DNA
   <213> Ectocarpus siliculosus
<400> 2
   gttctcgagc gcatctacgg caactcgcgc atcggcagcc gctacttcgc cgtgccggac      60
   ttcacccccg gcagggcggc caagggcgac cccctcttct acccggccga cggcagctac     120
   caggtgcccg tggacgtccg gctggacaag ttcaaggaga aggccgtccc gctggtgtcc     180
   gacgtcgccc gccgcgccat caaggaggcc ggcctgaacg tcgaggacat ctccaagctc     240
   gtcgtggtct cctccaccgg attcctcggc cccggcctcg actgcgagct gatcaagaac     300
   ctcggcctga cccgctccgt cgaccgcacc ctcatcgggt tcatgggctg cgccgccgcc     360
   atgaacggtt tccgtaacgc gaacgactac gtcaccgcca accccggaaa gtacgcgctg     420
   atgatctgcg tcgagctttc ctcggtgcac acgacctttg acgacaacat caacgacgcg     480
   atcttgcacg ctatcttcgc cgacggatgc gccgctgccg tgctcaaggg agccaggaag     540
   tcggagtgcc ccaagggaac cctcgctatc gtcgacaacc acgcgtggct catggaggga     600
   accgaggacg gaatcaccct tgctatcaag ccaaacggca tcacctgcac cctgtccaag      660
   ttcctgcccc agtacatcgc caagaacatc gccttcttcg cggacggctt cctcaagaag      720
   cacaagctcg gacgcgacga cgttgacttc tggtgcgtgc accccggagg ccgacgtatc      780
   atcgaggagg cgcagaacgg ccttggcctc tcggaggagc agaccgcgga ctcgtgggcg      840
   gtgctcgggg agtacggaaa catgctctcg                                       870
<210> 3
   <211> 380
   <212> PRT
   <213> Ectocarpus siliculosus
<400> 3
   Ser Lys Asp Glu Gln Thr Val Tyr Pro Val Ile Ala Gly Met Ala Ile
   1               5                   10                  15
   Gly Asn Pro Gln Tyr Arg Cys Thr Gln Asn Glu Ala Leu Ala Val Ala
               20                  25                  30
   Ser Lys Cys Pro Gly Leu Glu Ser Ile Lys Pro Val Leu Glu Arg Ile
           35                  40                  45
   Tyr Gly Asn Ser Arg Ile Gly Ser Arg Tyr Phe Ala Val Pro Asp Phe
       50                  55                  60
   Thr Pro Gly Arg Ala Ala Lys Gly Asp Pro Leu Phe Tyr Pro Ala Asp
   65                  70                  75                  80
   Gly Ser Tyr Gln Val Pro Val Asp Val Arg Leu Asp Lys Phe Lys Glu
                   85                  90                  95
   Lys Ala Val Pro Leu Val Ser Asp Val Ala Arg Arg Ala Ile Lys Glu
               100                 105                 110
   Ala Gly Leu Asn Val Glu Asp Ile Ser Lys Leu Val Val Val Ser Ser
           115                 120                 125
   Thr Gly Phe Leu Gly Pro Gly Leu Asp Cys Glu Leu Ile Lys Asn Leu
       130                 135                 140
   Gly Leu Thr Arg Ser Val Asp Arg Thr Leu Ile Gly Phe Met Gly Cys
   145                 150                 155                 160
   Ala Ala Ala Met Asn Gly Phe Arg Asn Ala Asn Asp Tyr Val Thr Ala
                   165                 170                 175
   Asn Pro Gly Lys Tyr Ala Leu Met Ile Cys Val Glu Leu Ser Ser Val
                   180                 185                 190
   His Thr Thr Phe Asp Asp Asn Ile Asn Asp Ala Ile Leu His Ala Ile
           195                 200                 205
   Phe Ala Asp Gly Cys Ala Ala Ala Val Leu Lys Gly Ala Arg Lys Ser
       210                 215                 220
   Glu Cys Pro Lys Gly Thr Leu Ala Ile Val Asp Asn His Ala Trp Leu
   225                 230                 235                 240
   Met Glu Gly Thr Glu Asp Gly Ile Thr Leu Ala Ile Lys Pro Asn Gly
                   245                 250                 255
   Ile Thr Cys Thr Leu Ser Lys Phe Leu Pro Gln Tyr Ile Ala Lys Asn
               260                 265                 270
   Ile Ala Phe Phe Ala Asp Gly Phe Leu Lys Lys His Lys Leu Gly Arg
           275                 280                 285
   Asp Asp Val Asp Phe Trp Cys Val His Pro Gly Gly Arg Arg Ile Ile
       290                 295                 300
   Glu Glu Ala Gln Asn Gly Leu Gly Leu Ser Glu Glu Gln Thr Ala Asp
   305                 310                 315                 320
   Ser Trp Ala Val Leu Gly Glu Tyr Gly Asn Met Leu Ser Pro Ser Val
                   325                 330                 335
   Met Phe Val Leu Ser Arg Val Phe Lys Arg His Asn Ala Ala Leu Ala
               340                 345                 350
   Gln Gly Lys Pro Gly Tyr Gln Thr Gly Met Ala Phe Ser Phe Ser Pro
           355                 360                 365
   Gly Val Gly Ala Glu Gly Ile Leu Leu Arg Gln Ile
       370                 375                 380
<210> 4
   <211> 1143
   <212> DNA
   <213> Ectocarpus siliculosus
<400> 4
   tccaaggacg agcagacggt atacccggtc atcgccggga tggccatcgg caacccgcag 60
   taccgctgca cgcagaacga ggccctcgcc gtcgcctcca agtgcccggg cctcgagtcc 120
   atcaagcccg ttctcgagcg catctacggc aactcgcgca tcggcagccg ctacttcgcc 180
   gtgccggact tcacccccgg cagggcggcc aagggcgacc ccctcttcta cccggccgac 240
   ggcagctacc aggtgcccgt ggacgtccgg ctggacaagt tcaaggagaa ggccgtcccg 300
   ctggtgtccg acgtcgcccg ccgcgccatc aaggaggccg gcctgaacgt cgaggacatc 360
   tccaagctcg tcgtggtctc ctccaccgga ttcctcggcc ccggcctcga ctgcgagctg 420
   atcaagaacc tcggcctgac ccgctccgtc gaccgcaccc tcatcgggtt catgggctgc 480
   gccgccgcca tgaacggttt ccgtaacgcg aacgactacg tcaccgccaa ccccggaaag 540
   tacgcgctga tgatctgcgt cgagctttcc tcggtgcaca cgacctttga cgacaacatc 600
   aacgacgcga tcttgcacgc tatcttcgcc gacggatgcg ccgctgccgt gctcaaggga 660
   gccaggaagt cggagtgccc caagggaacc ctcgctatcg tcgacaacca cgcgtggctc 720
   atggagggaa ccgaggacgg aatcaccctt gctatcaagc caaacggcat cacctgcacc 780
   ctgtccaagt tcctgcccca gtacatcgcc aagaacatcg ccttcttcgc ggacggcttc 840
   ctcaagaagc acaagctcgg acgcgacgac gttgacttct ggtgcgtgca ccccggaggc 900
   cgacgtatca tcgaggaggc gcagaacggc cttggcctct cggaggagca gaccgcggac 960
   tcgtgggcgg tgctcgggga gtacggaaac atgctctcgc cctccgttat gttcgttctg 1020
   tctagggttt tcaagcgcca caacgccgcg ctcgcacagg gcaagcccgg ctaccagacg 1080
   ggtatggcgt tctccttctc gccgggtgtc ggggcagagg gcatccttct caggcagatc 1140
   tag 1143
<210> 5
   <211> 102
   <212> DNA
   <213> Ectocarpus siliculosus
<400> 5
   atgtcttctg ctgcggttgc tatgctggct gacccgactg tccagatcgc tctggcgtgc 60
   ctggtggtgt ctctcttcgt tgtgctgcag tcggtcaaaa ag 102
<210> 6
   <211> 34
   <212> PRT
   <213> Ectocarpus siliculosus
<400> 6
   Met Ser Ser Ala Ala Val Ala Met Leu Ala Asp Pro Thr Val Gln Ile
   1               5                   10                  15
   Ala Leu Ala Cys Leu Val Val Ser Leu Phe Val Val Leu Gln Ser Val
               20                  25                  30
   Lys Lys
<210> 7
   <211> 6
   <212> PRT
   <213> Artificial Sequence
<220>
   <223> Séquence étiquette
<400> 7
   His His His His His His
   1               5
<210> 8
   <211> 18
   <212> DNA
   <213> Artificial Sequence
<220>
   <223> Séquence étiquette
<220>
   <221> misc_feature
   <222> (3)..(3)
   <223> y = c ou t
<220>
   <221> misc_feature
   <222> (6).. (6)
   <223> y = c ou t
<220>
   <221> misc_feature
   <222> (9)..(9)
   <223> y = c ou t
<220>
   <221> misc_feature
   <222> (12)..(12)
   <223> y = c ou t
<220>
   <221> misc_feature
   <222> (15)..(15)
   <223> y = c ou t
<220>
   <221> misc_feature
   <222> (18)..(18)
   <223> y = c ou t
<400> 8
   caycaycayc aycaycay 18
<210> 9
   <211> 12
   <212> PRT
   <213> Artificial Sequence
<220>
   <223> Séquence étiquette
<400> 9
   Met Arg Gly Ser His His His His His His Gly Ser
   1               5                   10
<210> 10
   <211> 36
   <212> DNA
   <213> Artificial Sequence
<220>
   <223> Séquence étiquette
<400> 10
   atgcgcggca gccatcatca tcatcatcat ggcagc 36
<210> 11
   <211> 54
   <212> DNA
   <213> Artificial Sequence
<220>
   <223> Amorce de clonage
<400> 11
   ggcggatccg catgcatgtc caaggacgag cagacggtat acccggtcat cgcc 54
<210> 12
   <211> 46
   <212> DNA
   <213> Artificial Sequence
<220>
   <223> Amorce de clonage
<400> 12
   ggctaagctt ttactagatc tgcctgagaa ggatgccctc tgcccc 46